(19) Europäisches Patentamt / European Patent Office / Office européen des brevets

(11) **EP 3 475 420 B1**

(12) **EUROPEAN PATENT SPECIFICATION**

(45) Date of publication and mention
of the grant of the patent:
**16.07.2025 Bulletin 2025/29**

(21) Application number: **17735038.6**

(22) Date of filing: **23.06.2017**

(51) International Patent Classification (IPC):
**C12N 9/48** $^{(2006.01)}$   **C11D 3/386** $^{(2006.01)}$
**A23K 20/189** $^{(2016.01)}$   **A23K 50/00** $^{(2016.01)}$
**A61K 38/48** $^{(2006.01)}$   **A23J 3/00** $^{(2006.01)}$

(52) Cooperative Patent Classification (CPC):
**A23K 20/189; A23J 3/00; A23K 50/00;
A23K 50/30; A23K 50/40; A23K 50/75;
A23K 50/80; C12N 9/16;** C12Y 301/03008

(86) International application number:
**PCT/EP2017/065529**

(87) International publication number:
**WO 2017/220776 (28.12.2017 Gazette 2017/52)**

(54) **FEED COMPOSITION COMPRISING AN ACID PROTEASE**

FUTTERMITTELZUSAMMENSETZUNG ENTHALEND EINE SAURE PROTEASE

COMPOSITION ALIMENTAIRE COMPRENANT UNA PROTÉASE ACIDE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **23.06.2016 EP 16176044
29.09.2016 GB 201616555**

(43) Date of publication of application:
**01.05.2019 Bulletin 2019/18**

(73) Proprietor: **EW Nutrition GmbH
49429 Visbek (DE)**

(72) Inventors:
• **MICHELS, Andreas**
  **40237 Düsseldorf (DE)**
• **SCHEIDIG, Andreas**
  **26810 Westoverleding (DE)**
• **MARTINEZ-MOYA, Ronny**
  **172047 La Serena (CL)**
• **WEBER, Diana**
  **40764 Langenfeld Rhl. (DE)**

(74) Representative: **Michalski Hüttermann & Partner
Patentanwälte mbB
Kaistraße 16A
40221 Düsseldorf (DE)**

(56) References cited:
**WO-A1-2016/062855**   **WO-A1-2016/062857**
**US-A- 4 473 644**

• **U. REICHARD ET AL: "Sedolisins, a New Class of
Secreted Proteases from Aspergillus fumigatus
with Endoprotease or Tripeptidyl-Peptidase
Activity at Acidic pHs", APPLIED AND
ENVIRONMENTAL MICROBIOLOGY, vol. 72, no.
3, 1 March 2006 (2006-03-01), pages 1739 - 1748,
XP055000173, ISSN: 0099-2240, DOI: 10.1128/
AEM.72.3.1739-1748.2006**
• **K. ODA: "New families of carboxyl peptidases:
serine-carboxyl peptidases and glutamic
peptidases", JOURNAL OF BIOCHEMISTRY, vol.
151, no. 1, 19 October 2011 (2011-10-19), pages
13 - 25, XP055143022, ISSN: 0021-924X, DOI:
10.1093/jb/mvr129**
• **GIULIANA CATARA ET AL: "A new kumamolisin-
like protease from Alicyclobacillus
acidocaldarius : an enzyme active under extreme
acidic conditions", BIOCATALYSIS AND
BIOTRANSFORMATION., vol. 24, no. 5, 1 January
2006 (2006-01-01), CH, pages 358 - 370,
XP055367483, ISSN: 1024-2422, DOI: 10.1080/
10242420600792094**

EP 3 475 420 B1

**(Cont. next page)**

- JIANZHUANG YAO ET AL: "Understanding the Autocatalytic Process of Pro-kumamolisin Activation from Molecular Dynamics and Quantum Mechanical/Molecular Mechanical (QM/MM) Free-Energy Simulations", CHEMISTRY - A EUROPEAN JOURNAL, vol. 19, no. 33, 12 August 2013 (2013-08-12), pages 10849 - 10852, XP055367440, ISSN: 0947-6539, DOI: 10.1002/chem.201301310
- WLODAWER ET AL: "How to Kill an Enzyme (In More Ways Than One)", STRUCTURE, ELSEVIER, AMSTERDAM, NL, vol. 12, no. 7, 1 July 2004 (2004-07-01), pages 1117 - 1119, XP025917191, ISSN: 0969-2126, [retrieved on 20040713], DOI: 10.1016/J.STR.2004.06.003
- NORIO SUZUKI ET AL: "Grifolisin, a member of the sedolisin family produced by the fungus Grifola frondosa", PHYTOCHEMISTRY, vol. 66, no. 9, 26 April 2005 (2005-04-26), GB, pages 983 - 990, XP055367594, ISSN: 0031-9422, DOI: 10.1016/j.phytochem.2005.02.014
- KENJI TAKAHASHI ET AL: "Structural Evidence That Scytalidolisin (Formerly Scytalidopepsin A) Is a Serine-Carboxyl Peptidase of the Sedolisin Family", BIOSCIENCE BIOTECHNOLOGY BIOCHEMISTRY., vol. 72, no. 8, 23 August 2008 (2008-08-23), TOKYO, JAPAN, pages 2239 - 2242, XP055367516, ISSN: 0916-8451, DOI: 10.1271/bbb.80258
- ODA KOHEI ED - NEIL D RAWLINGS ET AL: "Kumamolisin", HANDBOOK OF PROTEOLYTIC ENZYMES : VOLUME 1 (THIRD, ACADEMIC PRESS, UK, 1 January 2013 (2013-01-01), pages 3339 - 3344, XP008184371, ISBN: 978-0-12-382219-2, DOI: 10.1016/B978-0-12-382219-2.00737-7
- NAKAYAMA TORU ED - NEIL D RAWLINGS ET AL: "Kumamolisin-As", HANDBOOK OF PROTEOLYTIC ENZYMES : VOLUME 1 (THIRD, ACADEMIC PRESS, UK, 1 January 2013 (2013-01-01), pages 3344 - 3347, XP008184372, ISBN: 978-0-12-382219-2, DOI: 10.1016/B978-0-12-382219-2.00738-9
- BYUNG RHO ET AL: "Aorsin, a novel serine proteinase with trypsin-like specificity at acidic pH", BIOCHEM. J, vol. 371, 1 January 2003 (2003-01-01), pages 541 - 548, XP055404238

Remarks:

The complete document including Reference Table(s) and the Sequence Listing(s) can be downloaded from the EPO website

**Description**

**Field of the invention**

The present invention relates to the field of proteases for feedstuff and other industrial applications.

**Background**

Proteases play an important role in different sectors of industry, including animal feed and care, fruit and beverage processing, and others (detergent, leather processing, production of protein hydrolysates, hard surface cleaning or biofilm cleaning treatment of necrotic or burned tissue to promote wound healing, to name a few).

The proteases commonly used are mainly serine proteases belonging to the peptidase families S1, e.g. the chymotrypsin family, or the subtilisin family S8, e.g., keratinase from *Bacillus.* These enzymes and others of these families have a pH optimum in the neutral or basic range.

In some cases enzymes are administered as zymogens, which is beneficial under certain conditions due to increased thermal, proteolytic and process stability. It has been described that activation of the zymogen to the active enzyme is a cumbersome process or activation under the process conditions may be slow. See, e.g. US 9,017,667, in which the zymogen of cysteine endoprotease (EP) from barley is provided in an acidic formulation comprising an acid or acidic buffer system, to ensure rapid activation when the zymogen is administered to a subject oral administration.

Yet, the pH optimum of these enzymes or the performance is not always suitable for the respective application.

It is hence one object of the present invention to improve the processability of proteases in the course of an industrial application.

It is hence one object of the present invention to provide protease compositions which have a better performance.

It is another object of the present invention to provide protease compositions which are more suitable to specific applications.

**Summary of the invention**

These and further objects are met with methods and means according to the independent claims of the present invention. The dependent claims are related to specific embodiments.

**Embodiments of the invention**

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Provided herein is a feed composition comprising a protease from peptidase family S53 is provided. Use of an S53 family protease in a feed composition has previously been described in WO2016/062855 and WO2016/062857.

As used herein, the term "feed composition" relates to a feed additive, feed ingredient, feed supplement, and/or feedstuff for life stock and companion animals, comprising a protease from peptidase family S53.

Alternatively, the use of a protease from peptidase family S53 in a feed composition is provided. Similar to the feed composition as such, this embodiment can be combined with all embodiments set forth in the claims as dependent embodiments of the feed composition as such.

The inventors have shown that proteases from the S53 family increase feed efficiency much better than the most commonly used protease for use in feed, Ronozyme ProAct ("RPA"), distributed by DSM. This enzyme relies upon a serine protease of the S1 family of peptidases from the strain Nocardiopsis, it has maximum activity in neutral to alkaline conditions.

Compared to the latter, a feed composition comprising a protease from the S53 family demonstrated, *inter alia,*

- an increase in food conversion rate of about 89 % (see table 3 below, "Points FCR increase" 3.5 -> 6.6), and
- an increase in ileal digestibility of about 84 % (see table 4 below, "Averaged apparent ileal digestibility increase % over control" 4.3 -> 7.9)

This tremendous increase in efficacy was completely unprecedented, and came very surprising. It underlines that the use of S53 proteases in feed applications provides considerable potential.

S53 refers to the protease family of sedolisins, which are acid proteases. The term "sedolisin" refers to acid proteases from peptidase family S53 (MEROPS, see also Wlodawer et al, 2003). Both terms can be used interchangeably. Sedolisins comprise acid-acting endopeptidases and a tripeptidyl-peptidase. Sedolisins are endopeptidases with acidic pH optima that differ from the majority of endopeptidases in being resistant to inhibition by pepstatin (Oda et al., 1998) Crystal structures have shown that many pepstatin-insensitive carboxyl proteinases are either glutamic peptidases in family G1 or sedolisins in family S53, both groups being unrelated to the aspartic peptidases of clan AA that are inhibited by pepstatin.

The activation of sedolisins involves autocatalytic cleavage at low pH 3.5 which releases one or more peptides to deliver the maturated and active form. Said autocatalytic cleavage is inhibited under alkaline, neutral and lightly acidic conditions.

Sedolisins comprise a catalytic triad with Glu, Asp and Ser (E295, D299 and S502, numbering as in the alignment), and there is an additional Asp (D385) in the oxyanion hole. The Ser residue is the nucleophile equivalent to Ser in the catalytic triad Asp, His, Ser triad of subtilisin, and the Glu of the triad is a substitution for the His general base in subtilisin. The residue that orients the general base side chain is quite different between the families, however, being Asp299 in family S53 (closely following Glu295 in the sequence), in contrast to Asp137 preceding His in the sequence of subtilisin. Asp385 of the oxyanion hole corresponds to Asn259 in subtilisin. See the following table for an overview:

|  | Catalytic triad | | | Oxyanion hole | Residue that orients general base side chain | pH optimum |
|---|---|---|---|---|---|---|
| Sedolisin | E295 | D299 | S502 | D385 | D299 | acidic |
| Subtilisin | D137 | H168 | S325 | N259 | D137 | neutral |

The protein folds of sedolisins are clearly related to that of subtilisin, and both groups are sometimes called serine proteases. However, sedolisins have additional loops and their amino acid sequences are not closely related to subtilisins. Taken together with the quite different active site residues and the resulting lower pH for maximal activity, justifies the classification in separate protease families.

According to one embodiment of the invention, said S53 protease is at least one selected from the list consisting of:

| Type | MEROPS ID |
|---|---|
| Sedolisin | S53.001 |
| Sedolisin-B | S53.002 |
| Kumamolisin | S53.004 |
| Kumamolisin-B | S53.005 |
| Aorsin | S53.007 |
| Kumamolisin-AS (also called Kumamolisin 1) | S53.009 |
| Kumamolisin-AC | S53.009 |
| Grifolisin | S53.010 |
| Scytalidolisin | S53.011 |

In specific embodiments, said S53 protease is at least one selected from the group consisting of Kumamolisin-AS (also called Kumamolisin 1), Kumamolisin-AC and/or Grifolisin.

According to one embodiment of the invention, said composition further comprises a phytase, e.g., an acid or neutral active phosphatase active on inositol phosphates. Examples for such phytases are in EC classes 3.1.3.8 or 3.1.3.26 or 3.1.3.72.

A phytase (myo-inositol hexakisphosphate phosphohydrolase) is a phosphatase enzyme that catalyzes the hydrolysis of phytic acid (myo-inositol hexakisphosphate (IP6)), which is an organic form of phosphorus that is found in grains and oil seeds, or its lower phosphorylated partly dephosphorylated products, inositol penta to mono phosphate (IP5, IP4, IP3, IP2, IP) to release a usable form of inorganic phosphorus. Phytases have been found to occur in animals, plants, fungi and bacteria.

While no direct effects of proteases on phosphate release either from phytate or phosphorylated proteins are known and can not be expected from the nature and the catalytic mechanism of these enzymes, indirect effects based on the hydrolysis of protein phytate complexes might exist.

The publication of Selle et al. (2000) consolidates the information about phytate-protein complexes and discusses the effect of phytase and the phytase-associated positive effects on protein digestibility. Under acid conditions of the stomach, below the isoelectric point of proteins, binary protein-phytate complexes are formed, whereas ternary complexes of phytate, metal ions and proteins are formed at neutral pH (Cosgrove 1966, Anderson 1985). Binary protein-phytate complexes have been demonstrated *in vitro* at acid pH for several proteins e.g. glycinin (Okubo et al. 1976) the major protein in soybeans. The maximum of protein complexation to binary complexes have been described at pH 2 - 3 for globulin, with a dependence on the phytate to protein ratio. Rajendran & Prakash (1993) described progressive protein-protein aggregation after an initial binding of protein to phytate associated with conformational changes of the protein. Such aggregates might be recalcitrant to protein hydrolysis as several *in vitro* studies describe a reduction of peptic hydrolysis in the presence of phytate in acid conditions (Camus & Laporte, 1976). This initial description was extended by several studies consistently showing a reduction of peptic activity for plant storage and animal proteins (Kanaya et la. 1976; Inagawa et al. 1987; Knuckles et al. 1989, Vaintraub & Bulmaga 1991).

The working hypothesis was derived from these observations that the formation of binary protein-phytate complexes are a major aspect of the well-known anti-nutritive effect of phytate. The anti-nutritive effect of phytate today is only addressed by means of supplemented microbial phytase activity beside the effect of the liberation of dietary phosphate from phytate. The protein effect of phytase is most probably associated with the protein-phytate complexes, by hydrolysis of phytate to lower inositol phosphates with a rendered ability to form such complexes. It might also be that a new propagated reading of dosage recommendation, the superdosing of phytase exerts its effect by fast hydrolyzing phytate and interfering with the built up of such complexes. It is further described that phytase will hydrolyse soluble phytate better (Lonnerdal et al. 1989) than phytate in protein decorated complexes (Konishi et al. 1999, Bohn et al. 2007).

Releasing protein and phytate from such binary complexes, in which phytate and protein are both more recalcitrant to hydrolysis, by the action of a protease which can hydrolyze protein in such complexes or before such complexes are formed in parallel or before the phytase is active, does have the potential to have beneficial effects together with a phytase. From these observations there is a high potential that an acid protease able to hydrolyze protein in binary complexes or before such complexes have been formed will have beneficial effects on the digestibility of crude protein and phosphate in combination with endogenous or added phosphatases. Otherwise picking an acid enzyme is not obvious, as acid proteases do have less time to hydrolyze protein due to a 4 time longer retention time in the neutral parts of the gastrointestinal tract, the mostly lower thermal stability, fewer examples for the economical producibility and examples for genetic engineering of such enzymes for better performance.

Again, the inventors have shown that proteases from the S53 family, when used in combination with a phytase, increase feed efficiency much better than the most commonly used protease for use in feed, Ronozyme ProAct ("RPA").

Compared to a combination of RPA with a phytase, a feed composition comprising an acid protease from the S53 family and a phytase demonstrated, *inter alia,*

- an increase in food conversion rate of about 34 % (see table 5 below, "Points FCR increase" 13.9 -> 18.6), and
- an increase in phosphorous digestibility of about 117 % (see table 6 below, "Increase of phosphorus digestibility % over Quantum blue" 2.3 -> 5.0)

According to another aspect of the invention, a feed composition is provided comprising an acid protease from either the S53 family or the G1 family, and a phytase, e.g., an acid or neutral active phosphatase active on inositol phosphates. Examples for such phytases are in EC classes 3.1.3.8 or 3.1.3.26 or 3.1.3.72.

Acid proteases from the S53 family and the G1 family form a group of what were formerly termed "pepstatin-insensitive carboxyl peptidase". Initially, the pentapeptide pepstatin soon came to be thought of as a very general inhibitor of the endopeptidases that are active at acidic pH. Later, several acid-acting endopeptidases, namely from the S53 family and the G1 family, were found to be resistant to pepstatin. Hence, both families share a particular structural and/or functional relationship.

The term "acid protease", as used herein, refers to proteases that exhibit their maximum activity and stability in acid conditions (pH 2.0-5.0). By contrast, one of the most commonly used protease for use in feed is Ronozyme ProAct ("RPA"), manufactured by DSM. This enzyme relies upon a serine protease from the strain Nocardiopsis, it has maximum activity in neutral to alkaline conditions.

Typically, acid proteases are activated autocatalytically, for example by cleavage of one or more propeptides from the zymogen, thus delivering a matured, active enzyme. Said autocatalytic cleavage often occurs under acidic conditions, e.g., by acid hydrolysis of a respective peptide bond. Under non-acidic conditions, said cleavage is inhibited, thus keeping the proenzyme in its inactive form. Hence, activation can be controlled by merely adjusting the pH to a desired value.

Acid proteases are not well established in industrial use. In animal feed applications, neutral to alkali proteases are mainly used because in the small intestine, where proteases become active, an alkaline environment is predominant. To bypass the stomach, which usually has acidic conditions, such alkaline proteases are often provided in granules which persist the stomach passage and dissolve in the small intestine, hence protecting the alkali proteases encapsulated therein from denaturation.

Besides from the sedolisins as discussed above, such acid protease can be from the Peptidase family G1 (MEROPS Accession MER001320).

The enzymes are mostly secreted from cells as inactive proenzymes that activate autocatalytically at acidic pH. Said autocatalytic cleavage is inhibited under alkaline, neutral and lightly acidic conditions. The active site residues are Q107 and E190. It has further been interpreted that Glu136 is the primary catalytic residue. The most likely mechanism is suggested to be nucleophilic attack by a water molecule activated by the Glu136 side chain on the si-face of the scissile peptide bond carbon atom to form the tetrahedral intermediate. Electrophilic assistance, and oxyanion stabilization, are provided by the side-chain amide of Gln53.

In one embodiment, the acid protease from the Peptidase family G1 is at least one selected from the list consisting of:

| Type | MEROPS ID |
|---|---|
| Scytalidoglutamic peptidase | G01.001 |
| Aspergilloglutamic peptidase (Aspergillopepsin II, or A2) | G01.002 |
| PepG1 peptidase (Alicyclobacillus sp.) | G01.006 |

In this context, it is important to understand that phytases for industrial use, e.g., from EC classes 3.1.3.8 or 3.1.3.26 or 3.1.3.72, have their activity in the acid or, less often, neutral range. This is because phytate is insoluble in the alkaline to neutral range.

This theory, although not binding, might explain why the combination of an acid protease, as K1, with a phytase delivers a synergistic effect which exceeds the effect caused by a combination of the same phytase with the alkaline protease Ronozyme ProAct ("RPA").

Both can become active simultaneously, either already in a feedstuff that has a respective pH, or in a part of the animal's guts where acidic conditions are present. Hence, the acid protease can support the phytase-mediated phosphorous release by digesting binary protein-phytate complexes, hence making the complexed phytate readily available for the simultaneously acting phytase (see above).

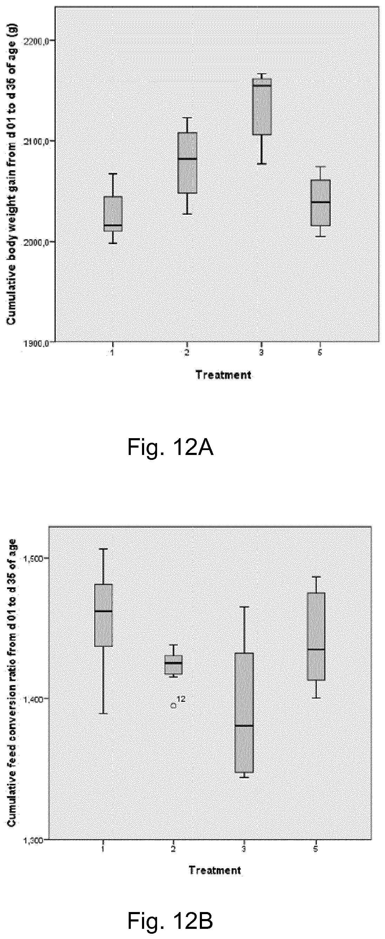

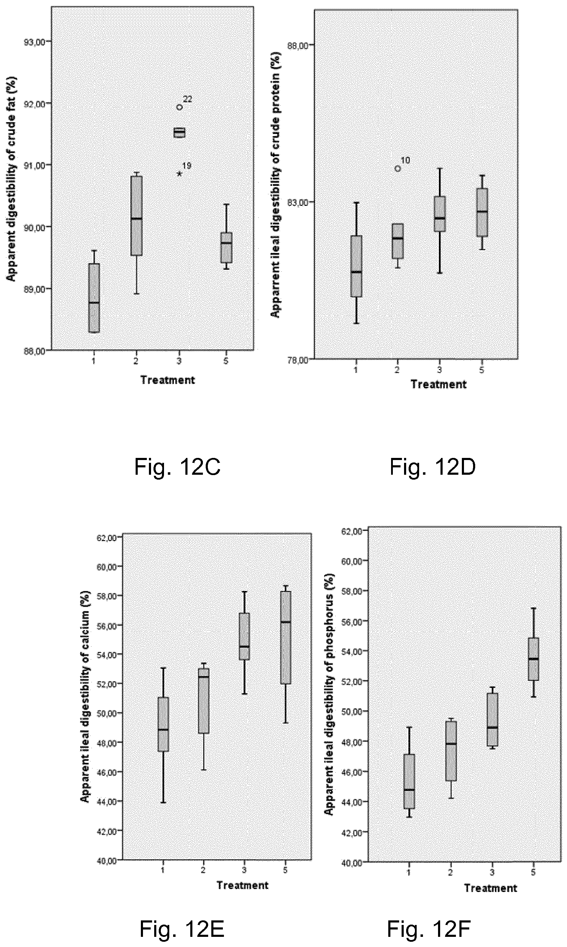

See again Figs. 12 - 13 and Tables 5 - 6, for experimental data on the said synergistic activity between an S53 protease and phytases, as compared to a combination of Ronozyme ProAct with a phytase. The same synergism applies for the combination of an acid protease from the G1 family, like aspergillopepsin II, with a phytase.

According to one embodiment of the invention, the feed composition has a pH of $\geq 6$.

Surprisingly, the inventors found that a composition in which an acid protease is kept at a pH of $\geq 6$ does not affect the activity of the protease as such.

Preferably, the pH of said composition is at a value of $\geq 6.1$, $\geq 6.2$, $\geq 6.3$, $\geq 6.4$, $\geq 6.5$, $\geq 6.6$, $\geq 6.7$, $\geq 6.8$, $\geq 6.9$, $\geq 7$, $\geq 7.1$, $\geq 7.2$, $\geq 7.3$, $\geq 7.4$, $\geq 7.5$, $\geq 7.6$, $\geq 7.7$, $\geq 7.8$, $\geq 7.9$ or $\geq 8$.

More preferably, the pH of said composition is at a value of $\geq 6.5$, or $\geq 7$.

Even more preferably, the pH of said composition is at a value of $\geq 7$.

The stability of enzymes under the conditions of production, storage and practical application is crucial to economically exploit the catalytic properties thereof in pharma, food, feed and other industrial or commercial applications. Proteases, also called peptidases, support the hydrolysis of proteins, by hydrolyzing peptide bonds of target proteins. Proteases can hydrolyze themselves or other proteins in a composition, including other enzymes. In nature, proteases are inhibited by peptide extensions typically called propeptides. These propeptides control the activity of proteases, with the zymogen, i.e., the unprocessed protease comprising the active enzyme core and the propeptide extension, being inactive. This mode of the so-called autoinhibition differs for different protease groups.

Surprisingly, the inventors found that a composition comprising an acid protease which is maintained at a pH as set forth above, i.e., under near-neutral, neutral or alkaline conditions, does not significantly affect the protease as such. At the same time, however, the protease is protected from autocatalytic activation, thus avoiding degradation of other enzymes in the composition, if present, and autocatalytic degradation, as well. Furthermore, the inventors found, surprisingly, that even under conditions of long term storage at the said near-neutral, neutral or alkaline conditions, the acid proteases can still be activated, e.g., by autocatalytic cleavage of the propeptide in an acid environment. Thus, their capacity of autoactivation remains unaffected even after long term storage under conditions that are unfavorable for acid proteases.

It is important to understand that feed additives, feed ingredients, feed supplements, or feedstuffs for animals are preferably stored under acidic conditions, or formulated in such way, to avoid microbial growth, including bacteria and fungi.

When conceiving the composition according to the present invention, the inventors overrode this teaching, and surprisingly found that a non-acidic composition, as discussed above, has beneficial effects, by avoiding degradation of other enzymes in the composition, if present, and autocatalytic degradation, as well. Furthermore, however, the inventors found that microbial growth can be kept at bay, too.

Preferably, the feed additive, ingredient, supplement or feedstuff is for at least one selected from the group consisting of monogastric species like poultry, pig, fish, companion animals and aquaculture.

In one embodiment, the acid protease is produced by homologous or heterologous protein expression.

In one further embodiment, the composition further comprises at least one agent or buffer that is present in a concentration suitable to maintain the pH of said composition at a value of $\geq 5$.

Suitable agents or buffers to establish such pH are, *inter alia,*

a) physiologically acceptable organic acids/salts, like citric acid/citrate, lactic acid/lactate, malic acid/malate, fumaric acid/fumarate acetic acid/acetate, glycine

b) physiologically acceptable inorganic acids/salts, like phosphoric acid/phosphate salts (like sodium phosphate or potassium phosphate), HCl/chloride salts (like calcium chloride).

In one other embodiment, the pH of $\geq 5$ is caused by the protein expression as such, or by the cultivation conditions or fermentation conditions. This embodiment encompasses, e.g., a composition in which the pH of $\geq 5$ is caused by fermentation with a host that establishes, by itself, pH conditions in the claimed range. This applies, *inter alia,* for *Bacillus* strains, which establish a pH of, e.g., 7.5 - 8 during fermentation, or other bacterial hosts, like *Streptomyces sp., Corynebacterium sp.,* or *E. coli.*

However, establishment of a pH by the protein expression as such, or by the cultivation conditions or fermentation conditions, can also depend on the type of energy source used during protein expression. If the energy source is largely protein-based, basic reaction products will drive the pH into a range > 5. Generally, during fermentation, the resulting pH can also be adjusted or controlled by suitable correction media.

The enzyme industry is a rather conservative one, which does not actually leave beaten tracks, e.g., for safety concerns or avoidance of bureaucratic approval procedures. As discussed above, proteases currently in use for feed applications are mainly serine proteases belonging to

(i) the peptidase families S1, e.g. the chymotrypsin family, or
(ii) the subtilisin family S8, e.g., keratinase from *Bacillus*

These enzymes and others of these families have a pH optimum in the neutral or basic range. However, as discussed, the inventors have realized that the use of an acid protease, like a sedolisin, has significant advantages in the described feed applications.

Hence, the use of an acid protease as or in a feed additive, feed ingredient, feed supplement, and/or feedstuff for life stock and companion animals is provided.

In the following, preferred embodiments regarding the acid protease will be described. It is important to understand that these preferred embodiments refer to both (i) the sedolisin enzyme as such, irrespective of a formulation or composition comprising the latter, let alone a pH thereof, and it's use in feed applications, as well as (ii) the composition comprising the sedolisin, having a pH of $\geq 5$.

In one embodiment of the invention, it is provided that the protease remains inactive at a pH of $\geq 6$ preferably, at a pH value of $\geq 6.1, \geq 6.2, \geq 6.3, \geq 6.4, \geq 6.5, \geq 6.6, \geq 6.7, \geq 6.8, \geq 6.9, \geq 7, \geq 7.1, \geq 7.2, \geq 7.3, \geq 7.4, \geq 7.5, \geq 7.6, \geq 7.7, \geq 7.8, \geq 7.9$ or $\geq 8$.

In one embodiment of the invention, the composition comprises at least one further enzyme. Preferably, said further enzyme is selected from the group consisting of cellulases (EC 3.2.1.91), xylanases (EC 3.2.1.8); arabinogalactan endo-beta-1,4-galactanase (EC 3.2.1.89); mannan endo-1,4-beta-mannosidase (EC 3.2.1.78), alpha-galactosidases (EC 3.2.1.22); phospholipase A1 (EC 3.1.1.32); phospholipases A2 (EC 3.1.1.4); lyso-phospholipases (EC 3.1.1.5); phospholipase C (3.1.4.3); phospholipase D (EC 3.1.4.4); amylases, beta-glucanases (EC 3.2.1.4 or EC 3.2.1.6), and/or neutral or alkaline active proteases.

The inventors have shown that the protective effect of the formulation according to the invention also extends on additional enzymes in the formulation, thus for example maintaining their activity during storage.

In a preferred embodiment of the invention, the composition or one or more enzymes therein has increased stability and/or storage life.

The composition has an increased stability, because under the conditions set forth the protease is protected from autocatalytic degradation (self-digestion). This again increases the storage life.

In case the composition comprises further proteins or even enzymes, as it may be the case, e.g., in a feed additive, a feed ingredient, a feed supplement, or a feedstuff for animals, these further proteins are likewise protected from digestion by the acid protease. This again increases the storage life, too.

In a preferred embodiment, the acid protease comprises one or more amino acid exchanges, insertions or deletions compared to the respective wildtype.

Preferably, the respective one or more exchanges, insertions or deletions serve to provide, to the acid protease, at least one of the features selected from the group consisting of

- increased activity
- increased thermostability
- optimized substrate specificity
- increased resistance against extreme pH values
- increased resistance or optimized performance in the presence of other feed ingredients
- increased resistance towards animals endogenous enzymes
- optimized producibility
- optimized activation speed
- increased thermal stability effects of propeptide, and/or
- optimized propeptide core enzyme interaction.

Increased thermostability is a particularly important feature as it makes the enzyme suitable for inclusion in premixes and pelleted feeds which undergo heat treatment.

According to another aspect of the invention, a method of activating a composition according to any one of the aforementioned claims is provided, which method comprises decreasing the pH of said composition to a value of ≤ 5 or smaller.

In one embodiment, the decrease of the pH is at least partly accomplished by

- adding a suitable agent or buffer to the composition,
- adding the composition to another composition that has a more acidic pH, and/or
- allowing the composition to decrease its pH by means of natural processes.

Said suitable agent or buffer can for example either be a physiologically acceptable organic acid/salt, like, for example, citric acid/citrate, lactic acid/lactate, malic acid/malate, fumaric acid/fumarate, acetic acid/acetate, acetic acid/acetate. It can also be a physiologically acceptable inorganic acid/salt, like, for example, phosphoric acid/phosphate salts (like sodium phosphate or potassium phosphate), HCl/chloride salts (like calcium chloride).

Said composition which has a more acidic pH can for example be a feedstuff that has an acidic pH, e.g., to reduce growth of bacteria, fungi, and the like.

The said natural processes can involve, *inter alia,* microbial growth, leading to an increase in $CO_2$, lactic acid, formic acid, acetic acid, butanoic acid, citric acid, oxalic acid, malic acid, succinic acid, propionic acid and/or protons by, e.g., proton ammonia ion antiport. All these processes contribute to a decrease in pH of the composition.

In one other embodiment, the decrease of the pH is at least partly accomplished *in situ* in the digestive tract of an animal. In this embodiment, it is the acidic pH in parts of the digestive tract of the animal which forages a feedstuff comprising the composition, that activates the acid protease therein.

In one other aspect of the invention, a feed additive, a feed ingredient, a feed supplement, or a feedstuff is provided which comprises the composition according to the invention. Feed additives, ingredients or supplements are also called "premix" sometimes.

In one further embodiment, the feed additive, ingredient, supplement, or feedstuff according comprises at least one agent selected from the group consisting of

- a fat-soluble vitamin,
- a water-soluble vitamin,
- a trace mineral, and/or

- an emulsifying agent.

Examples of fat-soluble vitamins are vitamin A, vitamin D3, vitamin E and vitamin K, e.g. vitamin K3. Examples of water-soluble vitamins are vitamin B12, biotin and choline, vitamin B1, vitamin B2, vitamin B6, niacin, folic acid and panthothenate, e.g. Ca-D-panthothenate, L-carnithin, pyrroloquinoline quinone (PQQ). Examples of trace minerals are manganese, zinc, iron, copper, iodine, selenium and cobalt.

Examples of emulsifying agents are those from the groups of glycolipids, as for example rhamnolipid or sophorolipids, or cholesterols, like cholic acids and the like.

In one further embodiment, a feedstuff (standard diet) comprising the feed additive, ingredient, or supplement is provided, which has a crude protein content of between $\geq 10$ and $\leq 500$ g/kg (1 - 50 % w/w).

Crude protein is calculated as nitrogen (N) multiplied by a factor 6.25, i.e. crude protein (g/kg) = N (g/kg) x 6.25. The nitrogen content is determined by the Kjeldahl method (A.O.A.C, 1984, Official Methods of Analysis 14th ed., Association of Official Analytical Chemists, Washington DC).

Preferably, the feedstuff (standard diet) has a crude protein content of between $\geq 50$ and $\leq 300$ g/kg (5 - 30 % w/w).

Typical crude protein contents of standard diets are shown in the following table:

| Animal | Typical crude protein content (w/w) |
| --- | --- |
| Pig | 13,5 - 21 % |
| Laying hen | 15 - 18,5 % |
| Turkey | 15,5 - 28 % |
| Broiler chicken | 16 - 23,5 % |
| Cow | 15 - 21 % |
| Horse | 8 - 16 % |

In such feedstuff, the added protease will contribute to a) increase the digestibility of the feedstuff, and b) increase of protein uptake from the feedstuff, hence improving animal health and nutrient uptake efficiency. It thus allows lower quality protein sources to be included in animal diets. The added protease will further optimize the gastrointestinal tract (GIT) microflora of the organism that forages on the feedstuff.

In one embodiment, said protein is digestible in the upper gastrointestinal tract and fermentable in the lower gastrointestinal tract of the subject.

In one embodiment, the feedstuff comprises the protease in an amount from $\geq 0.0005$ % to $\leq 0.5$ % w/w.

According to another aspect of the invention, a method of decreasing a population in the upper gastrointestinal tract is provided, the method comprising administering to the subject an acid protease or a composition according to the invention, wherein the population of bacteria is reduced in the upper gastrointestinal tract of the subject. The method of the invention is especially useful for this application as the acid proteases used herein hydrolyze proteins before entering the intestine. In one embodiment thereof, the population of bacteria comprise *Clostridium perfringens.*

According to another aspect of the invention, a method for improving feed efficiency is provided, which comprises modifying a standard diet to contain less protein, and supplementing the modified diet with at least an acid protease or composition according to the invention. Preferably, in that approach, the modified diet contains between $\geq 5$ % or $\leq 20$ % less protein than the standard diet. Such modified diet can be produced by replacing a mass of a protein supplement with an equivalent mass of a grain. Values for standard diets are given elsewhere herein.

According to another aspect a method of producing an acid protease by homologous or heterologous protein expression in a protein expression system is provided, in which method cultivation conditions are applied that lead to a pH of 5,5 or higher, for at least a given period of time, and at least locally.

In one embodiment of that method, the pH of the medium surrounding the protein expression system is

- established by addition of an agent or buffer that is present in a concentration suitable to maintain the pH of said composition at a value of $\geq 5$, and/or
- caused by the protein expression as such, or by the cultivation conditions or fermentation conditions.

The second embodiment encompasses, e.g., a composition in which the pH of $\geq 5$ is caused by fermentation with a host that establishes, by itself, pH conditions in the claimed range. This applies, *inter alia,* for *Bacillus* strains, *E. coli, Corynebacterium sp.* and *Streptomyces sp.,* which establish a pH of, e.g., 7.5 - 8 during fermentation.

Preferably, in said method the protein expression system is at least one selected from the group consisting of

- a yeast-based protein expression system
- a filamentous fungus-based protein expression system
- a bacterial protein expression system.

Preferably, the yeast-based protein expression system is selected from *Saccharomyces sp., Pichia sp., Hansenula sp.* and/or *Schizosaccharomyces sp., Arxula sp., Kluyveromyces sp..*

Preferably, the filamentous fungus-based protein expression system is selected from *Trichoderma sp., Aspergillus sp., Scytalidium sp., Grifola sp.,* and/or *Neurospora sp., Penicillium sp., Chrysosporium sp., Fusarium sp.* or *Myceliophthora sp..* Preferably, the bacterial protein expression system is selected from *Bacillus sp., Caulobacter sp., Lactococcus sp., Pseudomonas sp., Streptomyces sp., Corynebacterium sp.* and/or *E. coli.*

According to another aspect a method of screening for, or producing a protease with a particular stability against a given condition, or with a particular enzyme activity, is provided, which method comprises

> b) phenotypically characterizing individual members of a protease library for a given parameter, wherein at least part of the characterization is carried out under conditions which keep the protease in its deactivated state
> c) selecting one or more members of said library according to the outcome of the selection in step b), and, optionally
> d) isolating said one or more selected members.

In a preferred embodiment of this method and the following ones, the protease is an acid protease as defined above.

In one embodiment, the phenotypical characterization in step b) comprises the substeps of

> b1) pretreatment at a given temperature, and
> b2) subsequent measurement of protease activity.

Preferably, said pretreatment involves incubation of the protease at $\geq 60°C$ for $\geq 10$ mins. This leads, preferably, to a selection of genotypes with an optimized, i.e. increased, thermal stability.

In one embodiment, the method further comprises an initial step of

> a1) providing a library of proteases, and/or
> a2) producing a library of mutated proteases by mutagenesis of one or more genes or cDNA encoding for a given scaffold protease,

which step precedes step b).

Further, in one embodiment the method further comprises a subsequent step of e) producing said one or more members selected in step c), and optionally isolated in step d), by means of a suitable protein expression method.

In one embodiment thereof, in step b1), the protease is kept in its deactivated state by at least one step selected from

- establishing or keeping a medium pH of $\geq 5$,
- adding a peptide which mimics the propeptide and binds to the active site of the active protease
- adding a small molecule inhibitor which reversibly binds to the active site
- adding an aptamer or antibody binding to or blocking access to the active site with sufficiently high thermal stability
- providing a propeptide that consists, or comprises D-amino acids which can not be cleaved, or hydrolysed, from the protease under respectively applied conditions.

Again the protease is preferably an acid protease, from which the propeptide cannot be cleaved or hydrolysed under non-acid conditions.

As discussed above, the protease is preferably an acid protease. In this case, the preferred option, the pH of said medium is preferably established at any of $\geq 5.1, \geq 5.2, \geq 5.3, \geq 5.4, \geq 5.5, \geq 5.6, \geq 5.7, \geq 5.8, \geq 5.9, \geq 6, \geq 6.1, \geq 6.2, \geq 6.3, \geq 6.4, \geq 6.5, \geq 6.6, \geq 6.7, \geq 6.8, \geq 6.9, \geq 7, \geq 7.1, \geq 7.2, \geq 7.3, \geq 7.4, \geq 7.5, \geq 7.6, \geq 7.7, \geq 7.8, \geq 7.9$ or $\geq 8$.

In a further embodiment, the pretreatment at a given temperature is carried out in a medium that is characterized by at least one of the group consisting of

> b1) pH of $\geq 5$
> b2) added peptide which mimics the propeptide and binds to the active site of the active protease
> b3) added small molecule inhibitor which reversibly binds to the active site
> b4) added aptamer or antibody binding to or blocking access to the active site with sufficiently high thermal stability, and/or

b5) added propeptide that consists, or comprises D-amino acids which cannot be cleaved, or hydrolysed, from the protease under respectively applied conditions.

Again the protease is preferably an acid protease, from which the propeptide cannot be cleaved or hydrolysed under non-acid conditions.

This ensures that the protease is inactive during the pretreatment step, because the inhibitory propeptide remains associated to the protease. The pretreatment is thus carried out on the zymogen. Again, the protease is preferably an acid protease as defined above. In this case, the preferred option is pretreatment under pH of $\geq$ 5.

In such way, it can be avoided that prior or during the pretreatment, the protease activates itself and decreases it's own activity by self-digestion, thus leading to false results when the impact of the thermal pretreatment on protease activity is investigated. Further, a potential effect of the propeptide on thermostability is thus reflected in the subsequent measurements.

The inventors have shown that a respective screening to thermostability where the pretreatment is carried out with an activated protease, devoid of the propeptide or in the absence of reversible protease inhibitors, which are missing for these family of proteases, delivers false positives with a very high frequency, i.e. above 70 %.

According to further aspects the use of a protease from peptidase family S53 as a

- detergent
- fruit and beverage processing
- leather processing
- production of protein hydrolysates
- hard surface cleaning or biofilm cleaning
- treatment of necrotic or burned tissue to promote wound healing
- pharmaceutical use,
- processing aid in tissue engineering, and/or
- baking dough preparation

in a composition is provided.

In fruit and beverage processing, for example, the commonly used proteases having a pH optimum in neutral or alkaline range suffer from suboptimal performance, because the substrate to be processed is mostly acidic (fruit juices, mash, pomace, must, wort, beer, etc.). Here, the composition according to the present invention provides significant advantages, as the protease comprised can work under optimal conditions.

Proteases for feed or food have a higher chance to show synergistic effects with the endogenous proteolytic enzymes when active early in the gastrointestinal tract (GIT), where pH is acidic. Theses synergistic effects might result from early degradation of proteinaceous antimicrobial proteins or peptides before they can exert their negative effects in the GIT. These might be the breakdown of inhibitors of proteases and other enzymes secreted by the pancreas of monogastric animals.

For leather processing it had been shown that acid proteolytic processes, in this case using pepsin, are more gently than the conventionally used alkaline proteases and that they can act more efficient even at lower temperatures, optimizing the economics and environmental effects of this process.

The pharmaceutical use of the proteases can be manifold. It can be related to food uptake, but can also be related to a drug in its own right. The protease can for example be used for hydrolysis of unwanted toxic peptides or proteins in human or animal food. One specific example of such toxic peptides are gluten peptides, digestive proteolytic malfunction of which is the cause of celiac sprue or dermatitis herpetiformis. These peptides can be detoxified by means of the protease according to the present invention. Another example is beta-conglycinin, which is comprised, *inter alia,* in soy beans, and induces, *inter alia,* gut hypersensitivity.

Baking dough preparation is another useful field of use of the protease. Proteases added to a baking dough help to degrade the gluten protein comprised therein, thus helping to increase the dough's elasticity, and improving its quality. Because of microbial activity (sour dough lactobacilli or yeast) baking dough has oftentimes an acidic or near neutral pH. Furthermore, it is oftentimes desirable to keep the pH at an acidic or neutral range to inhibit amylases that degrade starch. Hence, acid proteases should preferably be used in such baking dough. The composition protects the acid protease during storage, and activation can either be accomplished by simply adding the composition to the already acidic baking dough, or lowering it's pH before adding it to the dough.

In one preferred embodiment, the composition has a pH of $\geq$ 5. The considerations and advantages discussed herein above apply *mutatis mutandis* for these embodiments.

**Experiments and Figures**

While the invention has been illustrated and described in detail in the drawings and foregoing description, such illustration and description are to be considered illustrative or exemplary and not restrictive; the invention is not limited to the disclosed embodiments. The invention is defined by the appended claims.

Short description of the Figures

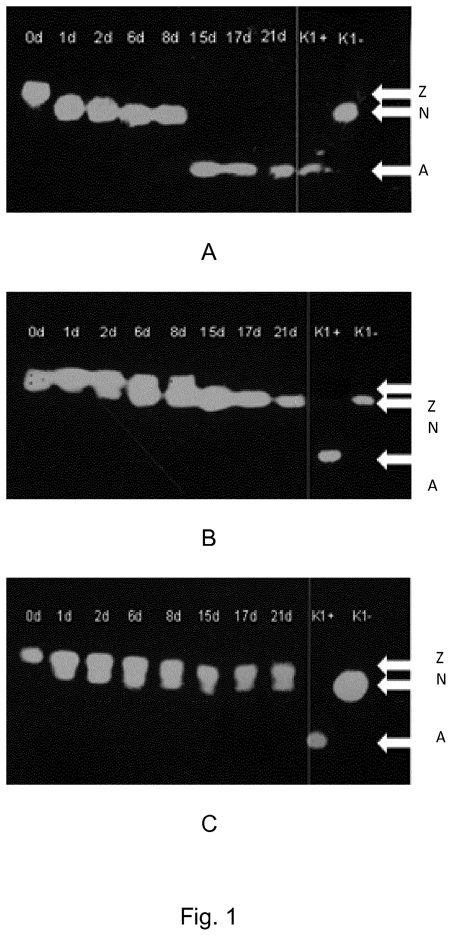

Fig. 1: Results of the Kumamolisin AS maturation experiments. Fig. 1A: pH 5.5, Fig. 1B: pH 6.0; Fig. 1C: pH 7.0

K1 +        Fully activated protease with processed propeptide
K1 -        Zymogen with nicked propeptide
0d to 21d   Samples stored for the indicated time at pH as outlined above. All samples were the unnicked zymogen at day 0 as checked by the thermal stability profile as outlined in example 5. Arrows indicate apparent mobility of enzyme species Z (zymogen form with propeptide unprocessed), N (the inactive enzyme with nicked propeptide) and A (the fully activated enzyme without propeptide)

After incubation above pH 7.0 the enzyme was not activated over an extended period of time. Only a fraction was initially nicked but there is no change over time and no activation at any time.
After incubation between pH 7.0 and pH 6.0 the enzyme is not activated but nicking of the enzymes proceeded over time until all enzyme was nicked. No activation is observed at any time.
After incubation below pH 6.0 the enzyme was nicked and activated over time with all enzyme being activated after 15 days and all enzyme being nicked within the first 24 hours.
Fig. 2: Zymogen of aspergilloglutamic protease, and active processed form, according to pH conditions. The zymogen has ~ 35 kDa, and the active processed form has ~ 30 kDa.
Fig. 3: Effect of pH and storage time on the thermal stability mediated by the propeptide. The effect of pH and storage time on the thermal stability was tested at different storage pH and ambient temperature as also outlined in example 4. Fig. 3 shows the respective thermostability curves after preincubation at the given pH values.
Fig. 4: Activation kinetics of Kumamolisin AS stored at different pH. Enzymes that were activated (stored at a pH of 5.5, time points t15, t17 and t21 at pH 5.5) showed a quick onset of activity, while enzymes stored under conditions where no activation, or nicking only, occurs (pH above 6.0, see also example 3 and 4) show a lag phase for activation of 3 minutes. No significant differences in activation lag time were observed for nicked or unprocessed zymogen.
Fig. 5: pH activity profile of activated and not activated Kumamolisin AS. The activated protease (e.g., in an acidic medium) showed a broader pH profile, being also active at pH values where only nicking but no activation occurs.

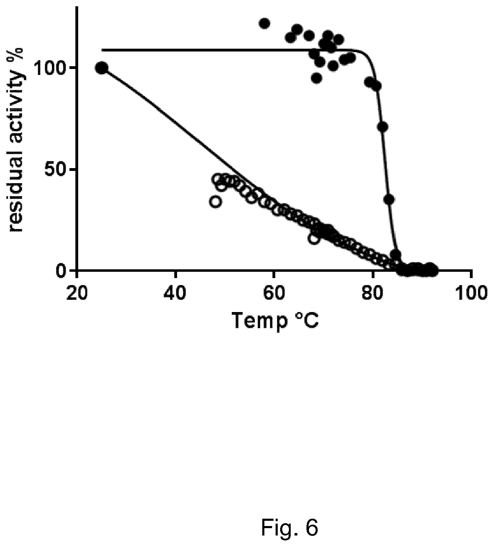

Fig. 6: Thermal stability of Kumamolisin AS for the zymogen (closed circles) or the activated enzyme (open circles). The thermal inactivation curve of the zymogen shows the standard sigmoidal inactivation curve, with a sharp decline in activity (curve can be fitted to a four parameter logistic) whereas the activated enzyme shows an early decline of activity as a mixed effect of self hydrolysis and thermal inactivation. Selecting protease variants for increased thermal stability with active or activated enzyme resulted in a high frequency of false positives due to this mixed effect. From 61 variants tested only 4 showed a slightly increased thermal stability, 5 showed wild type stability and 52 had reduced thermal stabilities.
Figs. 7 - 11: Results of experiment 10. Fig. 7 shows the body weight gain, Fig. 8 shows the feed conversion ratio, Fig. 9 shows the apparent digestibility of crude protein, Fig. 10 shows the apparent digestibility of fat, and Fig. 11 shows the apparent digestibility of phosphorous.
Fig. 12 A-F: Results of experiment 11. A: Body weight gain. B: Feed conversion ratio. C: Apparent ileal digestibility of fat. D: Apparent ileal digestibility of crude protein. E: Apparent ileal digestibility of calcium. F: Apparent illeal digestibility of phosphorous.
Fig. 13 A-F: Results of experiment 12. A: Body weight gain. B: Feed conversion ratio. C: Apparent ileal digestibility of crude protein. D: Apparent ileal digestibility of phosphorous. E: Apparent ileal digestibility of calcium. F: Apparent ileal digestibility of crude fat.
Fig. 14: Relative trypsin activities (%) on AAPF at pH 7 in the presence/absence of BBI/KTI and +/- hydrolysis of BBI/KTI with grifosilin. Black columns represent data with BBI/KTI hydrolysis with grifosilin, while white columns represents those without grifosilin treatment. All data are means from duplicates.

**Example 1: Production of enzymes**

### 1.1. Expression systems

The proteins were produced by means of heterologously expression in different host systems, depending on the source organism. Expression systems were:

Kumamolisin AS, as a reference for bacterial acid proteases of the protease family S53, was expressed in a *Bacillus subtilis* strain, a derivative of strain *Bacillus subtilis* 168. The codon usage optimized gene of Kumamolisin AS was expressed as a zymogen sequence (SEQ ID NO 1) from a plasmid and secreted into the culture medium.

Aspergilloglutamic peptidase, as a reference for acid proteases of the protease family G1 was expressed in *Hansenula polymorpha,* an auxotroph derivative of strain CBS4732. The codon usage optimized gene (SEQ ID NO 4) as expressed and secreted into the culture medium as the zymogen from a strain harboring a stable genomic integration of the gene.

### 1.2. Fermentation and preparation

Kumamolisin AS (K1): *Bacillus subtilis,* transformed with a plasmid harboring the codon optimized gene under the control of a constitutive promotor were cultivated in 1 L Erlenmeyer flasks containing 200 mL TB medium (12 g L$^{-1}$ trypton, 24 g L$^{-1}$ yeast extract, 1 % (w/v) glucose, 80 mM potassium phosphate, pH 7.2) supplemented with 20 $\mu$g mL$^{-1}$ neomycin. Cultivation was inoculated to an OD of 0.05 from a pre culture incubated on a rotary shaking table (150 rpm) at 37°C in 2 x Luria Bertani medium (20 g L$^{-1}$ pepton, 10 g L$^{-1}$ yeast extract, 5 g L$^{-1}$ NaCl, pH 7,5) supplemented with 20 $\mu$g mL$^{-1}$ neomycin. The medium was buffered with 200mM Tris/HCl at pH 7,5.

The expression culture was cultivated for 40 h at 37°C on a rotary shaking table at 150 rpm.

Aspergilloglutamic protease (A2): *Hansenula polymorpha* strains with stable genomic integrations of the codon optimized gene under the control of an inducible promotor were fermented in YNB synthetic medium with 2 % (w/v) glucose and 1 % (w/v) at a pH of 6.0.

Fermenter were inoculated to an OD of 1 from a pre culture run 20 h at 30°C on a rotary shaker at 130 rpm in 1 % (w/v) yeast extract, 2 % (w/v) peptone 2 % (w/v) glucose. The fermentation continued for 65 h at a cultivation temperature of 30°C. After onset of diauxy from glucose to glycerin, the cultivation was fed with 75 % Glycerin (w/v) 1,5-6 g L$^{-1}$h$^{-1}$, coupled to oxygen saturation as a set point. Further set points, air flow rate 2.5 L min$^{-1}$, stirrer speed 500 - 1500 rpm coupled to oxygen saturation. Correction solutions used for the control of pH and foam were, 33 % phosphoric acid (H$_3$PO$_4$), 12,5 % ammonia hydroxide (NH$_4$OH) and anti foam J6173.

### 1.3. Crude preparations

Cells were separated from crude culture broth of fermentations by centrifugation (17.000 rpm, 20 min, 4°C). Supernatants were decanted from the precipitate and filtered through 0,45 $\mu$m PES membrane in order to remove residual host cells.

The cell free crude fermentation supernatant was further concentrated 20 times on a crossflow membrane unit (Vivaflow 200, Hydrosart membrane, 10.000 Da cutoff). Initial concentrates were checked for pH which in all cases maintained the pH of the fermentation and were than further diafiltrated on the same crossflow system into different buffers, depending on the test condition. Buffers used for diafiltration were:

> pH 5,5 100 mM Na-Acetate buffer pH 5.5, 0.5 mM CaCl$_2$
> pH 6.0 100mM Na-Acetat buffer pH 6.0 + 0.5 mM CaCl$_2$
> pH 7.0 100mM HEPES buffer pH 7.0 + 0.5 mM CaCl$_2$

### Example 2: Protease assays and stability tests

#### 1.1. Protease assays

a) AAPF assay 96 well format

> Assay buffer: 200 mM sodium acetate or citrate, 1 mM CaCl$_2$, 0,01 % Triton X-100
> at pH 4 or pH 3 depending on the experiment
> Substrate stock solution: 100 mM in water free DMSO
> Substrate working solution: Substrate Stock solution diluted 1:50 in assay buffer, either pH 4 (acetate) or pH 3 (citrate)
> Execution: Load 50 $\mu$L of the diluted sample into the wells of a Nunc 96 clear flat bottom plate. Dilution is made in water containing 0.01 % Triton-X100 corresponding to the volumetric activity of the sample.

Start reaction by adding 50 μL of substrate working solution.
Measure kinetics at 37°C by monitoring the increase in adsorption at 410 nm as a measure for enzymatic activity.

b) $IT_{50}$: $IT_{50}$ defines the temperature where 50 % of the activity is inactivated under the conditions described above. Although not equivalent to, it is a measure for the thermal stability in the application, e.g. pelleting conditions or conditions in a detergent application, either dish washing or the cleaning of a fabric or hard surface and other technical applications.

Assay buffers:  50 mM sodium phosphate, 0,25 mM $CaCl_2$ pH 6.0
800 mM glycine pH 2.8

Thermal inactivation execution: Samples were diluted corresponding to the volumetric activity in potassium phosphate buffer. The pH of the final solution was checked to be above pH 5.5. The samples were transferred in replicates, 20 μL per well, into a 384 well PCR plate according the direction of the temperature gradient of the PCR machine. The plates were sealed with an adhesive or hot melting cover foil and incubated on a thermal gradient cycler with a temperature gradient of +/- 12°C around the expected IT50 value for 10 minutes. The samples were cooled to 8°C before measuring the residual activity of the samples with AAPF-pNA as followed. Samples, 15 μL each from the temperature incubation plate were transferred into a Nunc 384 clear flat bottom plate and 9 μL of glycine buffer were added to activate the protease during a incubation of 1 hour at 37°C. After the activation of the protease the assay was started by adding 24 μL of an AAPF-pNA solution (2 mM AAPF-pNA in water with 0.01 % Triton-X100) and activity was measured by following the kinetics at 37°C. The normalized experimental data for residual activity at the inactivation temperatures were fitted to a four parameter logistics function to evaluate the IT50.

**Example 3: Kumamolisin AS maturation of the propeptide tested by apparent gel mobility under native conditions**

Samples of the protease incubated at different pH were tested for the processing of the propeptide. pH was adjusted as follows:

| pH 5.5 | 100mM Na-Acetate buffer pH 5.5 + 0.1 mM $CaCl_2$ |
|---|---|
| pH 6.0 | 100mM Na-Acetate buffer pH 6.0 + 0.1 mM $CaCl_2$ |
| pH 7.0 | 100mM HEPES buffer pH 7.0 + 0.1 mM $CaCl_2$ |

Separation was performed on an native Gel (Mini-Protean TGX Stain free gel, any kD, Biorad 456-8126, with sample buffer 62.5 mM Tris pH 6.8, 12.5 % glycerol, Bromphenol-Blue, running buffer 25 mM Tris pH 8.5, 192 mM glycin). Gels were either stained (with coomassie or using the stainfree protocol of Biorad), or further analysed by a zymogram procedure. For zymograms the separated protease species were fully activated after separation in the gel, by rinsing the gel in water followed by an incubation in 100 mM sodium citrate pH 3.0 for 1h. Active bands were detected by lying an x-ray film (AGFA or Fuji, light exposed and developed, the gelatin side facing the gel) on top of the gel and incubating the gel for 30 minutes at 37°C in a humidified box. After incubation the x-ray film was rinsed with water to remove hydrolysed protein. Active bands are visual as translucent areas on a black film.
Three enzyme species can be distinguished based on the mobility, the zymogen (Z), the enzyme with nicked propeptide (N) and the processed, fully activated enzyme (A) (see Fig. 1).

**Example 4: pH dependent maturation of the aspergilloglutamic protease**

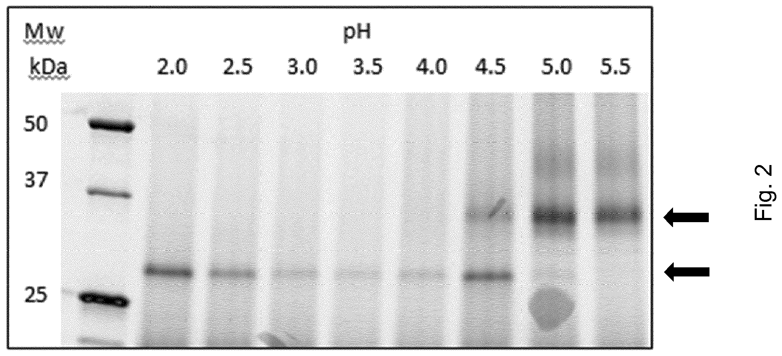

The pH that is required to activate the protease is backbone-dependent with Kumamolisin AS showing the highest pH. The pH for the activation of aspergilloglutamic protease is below 5.0 as deduced from the processing of the propeptide, judged by the shift in molecular weight of the zymogen (28,8 kDa theoretical) to the active processed form 23,1 kDa theoretical). Results from SDS-PAGE analysis are shown in Fig. 2.

**Example 5: Effect of pH and storage time on the thermal stability mediated by the propeptide**

The effect of pH and storage time on the thermal stability was tested at different storage pH and ambient temperature as also outlined in example 3. The thermal stability was tested at the indicated incubation times by analyzing the $IT_{50}$ value as described in example 2b.

Kumamolisin AS showed an apparent $IT_{50}$ of 83,5°C when not activated during preincubation (because of storage at a pH of 7), whereas the nicked and fully processed enzymes (i.e., which were activated during preincubation because of storage at a pH of 6 or lower) showed an $IT_{50}$ of 67,3°C.

The determination of $IT_{50}$ shows the ratio between inactive and active enzyme candidates. Enzyme candidates with higher stability are inactivated at higher temperatures ("right shift"), while enzyme candidates with lower stability are already inactivated at lower temperatures ("left shift"). Under activating conditions nicking occurs, or complete processing of the propeptide, respectively. This leads to the loss of the stabilizing effect of the propeptide. Hence, the processed enzyme candidate has a lower thermal stability ("left shift").

Nicking or activation hence produces a mixture of species, resulting in a biphasic thermal inactivation curve, with the inflection point giving the fraction of the stable not processed species. The transition from the nicked to the fully activated form results in a change of the slope of inactivation, as a mixed effect of thermal instability and auto proteolysis. Results from this experiment are following the quantitative analysis performed via apparent mobility in the native gel chromatography of example 3.

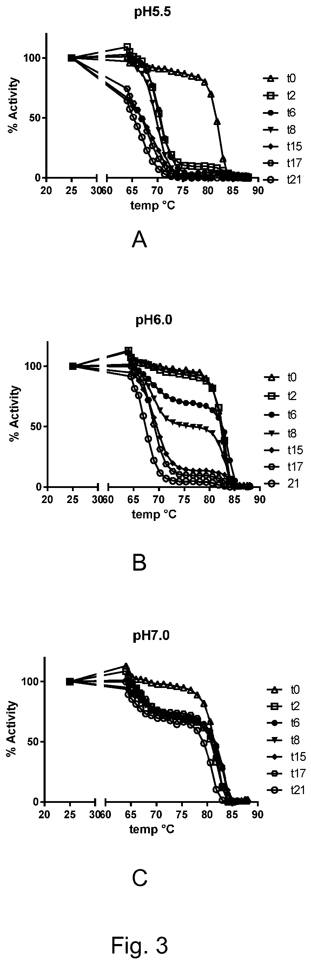

See Fig. 3 for the respective thermostability curves after preincubation at the given pH values 5.5; 6.0 and 7.0. t0 - t21 indicate the storage time/preincubation time in days. It is very obvious that at pH 5.5, only the sample that was not preincubated (storage time t0) had an $IT_{50}$ of higher than 80°C, while the samples preincubated for 1 day or more suffered an immediate loss in IT50 to lower temperatures.

Without being bound to theory, it appears that the point that the preincubation under activating conditions leads to a left shift in $IT_{50}$ is caused by hydrolysis of the propeptide, leading to a loss of the stabilising effect of the propeptide. Further, once the enzyme is fully activated, it becomes also self digesting.

### Example 6: Activation kinetics of Kumamolisin AS stored at different pH

The effect of storage time and pH on the activation state and activation kinetics was investigated at different storage pH and ambient temperature as also outlined in example 4. Activation kinetic was tested at the indicated time point by transferring the stored protease into an activity assay, as described in example 3, at a pH found in the stomach of monogastric animals.

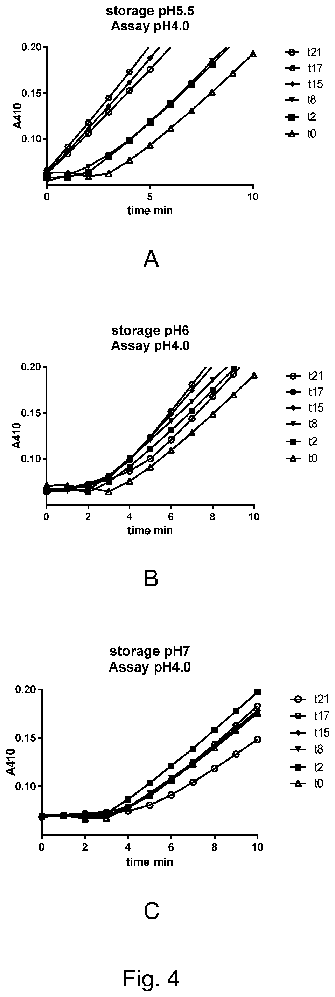

Fully activated samples hydrolyze proteins or protease substrate at maximum conversion speed without any lag time (time points t15, t17 and t21 at pH 5.5), whereas enzymes stored under conditions where no activation, or nicking only, occurs (pH above 6.0, see also example 3 and 4) show a lag phase for activation of 3 minutes. No significant differences in activation lag time were observed for nicked or unprocessed zymogen. Fast activation is beneficial as the time at acidic pH, where the protease of the invention can be active is limited. See Fig. 4 for results.

### Example 7: pH activity profile of activated and not activated Kumamolisin AS

A preparation of Kumamolisin AS produced under conditions where no activation occurs (i.e., at a pH > 6), was assayed for the activity at different pH using the assay described under example 3 but using the broad pH band assay buffer as described by Britton and Robinson (1931) without veronal.

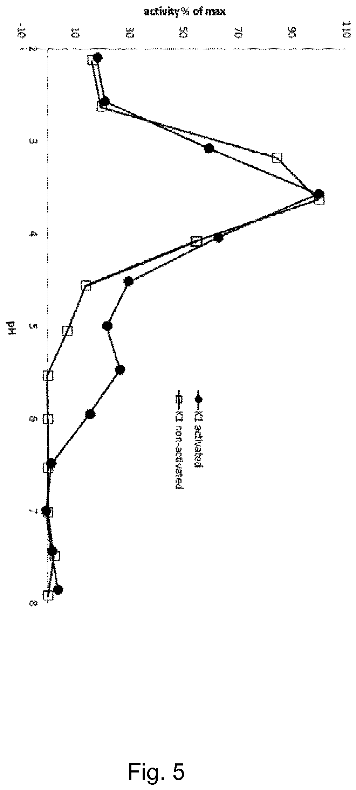

Activity was analysed at steady state conditions and activity normalized to the maximum activity observed. The activated protease showed a broader pH profile, being also active at pH values where only nicking but no activation occurs. See Fig. 5 for results.

### Example 8: Storage stability of feed enzymes in the presence of proteases

Besides having a positive effect on the thermal stability (see example 5), the propeptides of the zymogens also have an effect on the enzymatic activity of the protease, with the zymogen being inactive. This protects the protease from self-hydrolysis (see example 9) but also protects other enzymes in the medium (e.g., the feedstuff) from proteolytic degradation. Different enzymes used as feed additive were hence tested for degradation by the acid proteases Kumamolisin AS (K1) at pH where the acid proteases are not activated (pH 6) and at the pH where the protease is activated.

A commercial cellulase and a commercial phytase at 50 mg/L were incubated at different pH in the presence of the proteases dosed with equal activity, or the cellulose/phytase alone at the indicated pH values as control. The residual activity of the enzymes was tested at indicated time points using the following assays: Mix 20 µL of substrate solution (1 mM MU, methylumbelliferyl substrates, for cellulase and phytase in water) with 20 µL of the incubated enzyme mixtures, diluted corresponding to the expected activity, in a black 384 well plate. Incubate at 37°C for 30 minutes. Add 40 µL of a 500 mM sodium carbonate (pH 10.3) solution. Read the fluorescence 364 ex 448 nm and calculate the residual activity with respect to t0. Results are shown in table 1:

| commercial cellulase enzyme assayed at indicated time point with MUC | | |
|---|---|---|
| days stored | K1 pH4 | K1 pH 6 |
| 0 | ≥ 100 % | ≥ 100 % |
| 2 | ≥ 100 % | ≥ 100 % |
| 7 | 89 % | 93 % |
| 14 | ≥ 100 % | ≥ 100 % |
| 21 | 91 % | ≥ 100 % |
| 27 | ≥ 100 % | ≥ 100 % |

| commercial cellulase enzyme w/o protease | | | | |
|---|---|---|---|---|
| days stored | Ctrl pH 3 | Ctrl pH 4 | Ctrl pH 6 | Ctrl pH 8 |
| 0 | ≥ 100 % | ≥ 100 % | ≥ 100 % | ≥ 100 % |
| 2 | 92 % | ≥ 100 % | 98 % | ≥ 100 % |
| 7 | ≥ 100 % | 92 % | 97 % | 87 % |
| 14 | 95 % | ≥ 100 % | ≥ 100 % | ≥ 100 % |
| 21 | ≥ 100 % | 96 % | ≥ 100 % | ≥ 100 % |
| 27 | ≥ 100 % | ≥ 100 % | ≥ 100 % | ≥ 100 % |

**Table 1. Storage of commercial cellulose enzyme at different pH in the presence or absence of proteases, activated or as zymogen.**

| commercial phytase enzyme assayed at indicated time point with MUC | | |
|---|---|---|
| days stored | K1 pH4 | K1 pH 6 |
| 0 | 91 % | 96 % |
| 2 | 78 % | 92 % |
| 7 | 67 % | ≥ 100 % |
| 14 | 35 % | 89 % |
| 21 | 27 % | ≥ 100 % |
| 27 | 21 % | ≥ 100 % |

| commercial phytase enzyme w/o protease | | | | |
|---|---|---|---|---|
| days stored | Ctrl pH 3 | Ctrl pH 4 | Ctrl pH 6 | Ctrl pH 8 |
| 0 | 81 % | 78 % | 86 % | 89 % |
| 2 | 89 % | 89 % | 92 % | ≥ 100 % |
| 7 | 90 % | 98 % | 93 % | ≥ 100 % |
| 14 | ≥ 100 % | ≥ 100 % | 87 % | ≥ 100 % |
| 21 | ≥ 100 % | ≥ 100 % | ≥ 100 % | ≥ 100 % |
| 27 | ≥ 100 % | 89 % | ≥ 100 % | ≥ 100 % |

**Table 2. Storage of commercial phytase enzyme at different pH in the presence or absence of proteases, activated or as zymogen**

The data show clearly that coincubation with the acid protease at neutral or basic pH (which leaves the protease as the inactive zymogen) protects the other enzymes in the mixture (cellulase, phytase) from being digested, while coincubation with the acid protease at an acidic pH (which activates the protease by hydrolyzing the propetide) degrades the other enzymes in the mixture.

**Example 9: Selection of optimized variants with higher thermal stability**

Thermal stability of enzymes is a relevant parameter for technical enzymes in food, feed, detergent, cleaning und other applications. The thermal stability of enzymes can be optimized by means of directed evolution an expression describing a combination of generating a genetic diversity and functional selection of optimized, i.e. increased thermally stable variant enzymes. Functional screening of a genetic diversity under predictive conditions is essential. For proteases like those described herein screening for thermally more stable variants by methods as described in example 2b can be affected by the self hydrolysis of the protease.

Testing the thermal stability of Kumamolisin AS for the zymogen (Fig. 6, closed circles) or the activated enzyme (Fig. 6, open circles) characterize this difference. The thermal inactivation curve of the zymogen shows the standard sigmoidal inactivation curve, with a sharp decline in activity (curve can be fitted to a four parameter logistic) whereas the activated enzyme shows an early decline of activity as a mixed effect of self hydrolysis and thermal inactivation.

**Example 10: *In vivo* digestibility test of acid protease**

To test the efficacy of acid proteases in feed applications Kumamolisin AS ("K1"), as example for a peptidase of the sedolisin group was tested in an *in vivo* trial.

Enzyme produced as described in example 1 was freeze dried as active enzyme and incorporated in a standard corn soy diet at a dosage depending on the activity of the samples of 35 mg/kg for K1.

After a 21 day pre-treatment period where the diet was fed without enzyme, male broiler chickens (Cobb 500) were assigned to three treatments (24 animals per treatment, 8 repetitions with three birds per cage, base area 34 cm x 55 cm), and fed for further 7 days a basal diet without (control group) or with enzyme supplementation at the dose levels stated. Feed was offered in automatic feeders *ad libitum.* Fresh water in drinking quality was continuously supplied by nipple drinkers.

The efficacy was demonstrated by productive performance (body weight, body weight gain, feed intake, feed conversion ratio) from 22 to 28 d of age, and apparent ileal digestibility measurements (ash, crude protein, crude fat, calcium, phosphorus) at the end of the 7 d feeding period (28 d of age).

For calculation of the individual body weight gain the following formula was used:

*Average weight gain per bird for each period F - S (corrected by weight gain of died or culled chickens)*

    *F - Average weight of the live birds in the cage at the weighing day*
    *S - Average weight of the live birds in the cage at the previous weighing*

The feed intake (corrected for dispersed feed) was calculated by the following formula

$$Feed\ intake\ per\ period = \frac{total\ feed\ consumed\ per\ cage}{(number\ of\ surviving\ birds\ \times days\ of\ the\ period) + days\ of\ died\ birds\ alive}$$

The feed conversion ratio was estimated by using the following formula:

$$Feed\ conversion\ per\ period = \frac{total\ feed\ consumed\ per\ cage}{total\ weight\ gain\ for\ the\ period\ (with\ died\ or\ culled\ chicken)}$$

Apparent ileal digestibility was determined in all birds per treatment group at the end of the 7 d treatment period. The ileal contents of 3 birds of one cage were pooled. Before analysing the pooled samples were kept at - 20°C before being freeze-dried for chemical analyses. Titanium(IV) oxide (TiO2) was supplemented as an inert marker at the dose level of 3 g/kg diet from 22 to 28 days of age. For calculation of the apparent ileal digestibility the following formula was used:

$$Ileal\ digeatability\ (\%) = 100 - \left[\frac{\%\ marker\ in\ feed}{\%\ marker\ in\ ileum} \times \frac{\%\ nutrient\ in\ ileum}{\%\ nutrient\ in\ feed} \times 100\right]$$

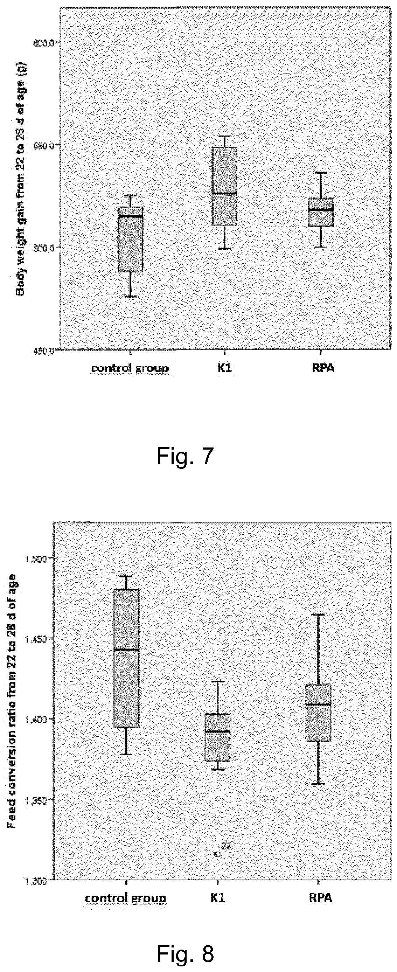

Results are shown in Figs. 7 - 11, and in tables 7 and 8.

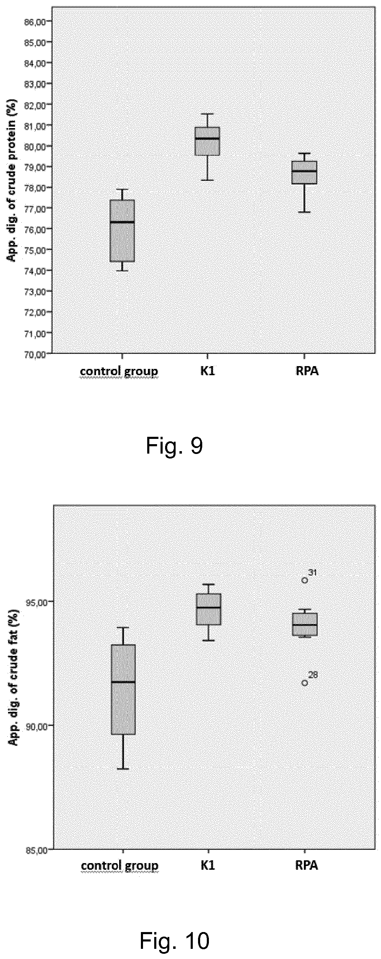

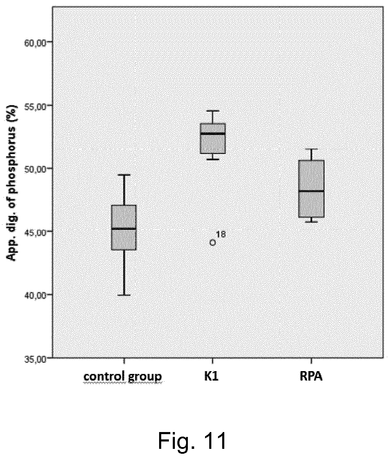

Fig. 7 shows the body weight gain, Fig. 8 shows the feed conversion ratio, Fig. 9 shows the apparent digestibility of crude protein, Fig. 10 shows the apparent digestibility of fat, and Fig. 11 shows the apparent digestibility of phosphorous.

It is indeed surprising that the digestibility of fat and phosphorous is increased by administration of the acid protease. This effect could not be expected, because proteases do not digest fat or phosphorous-comprising molecules. Without being bound to theory, the inventors speculate that the acid proteases might cleave up particular complexes comprising proteins, which then release fats or phosphorous.

These experiments show that the acid proteases according to the invention - in particular those from the sedolisin group - are indeed useful as feed additives, to increase digest-ability of food and body weight gain.

**Example 11: *In vivo* performance test**

To test the efficacy of acid protease Kumamolisin AS (K1) as an example for an acid protease of the group of sedolisins (S53), an *in vivo* trial against a "Ronozyme ProAct" (RPA) was carried out. As discussed, the latter enzyme is a protease with a neutral pH activity profile, and was included to demonstrate the performance advantage of said acid proteases of the group of sedolisins (S53), over neutral active proteolytic enzymes.

Enzyme produced as described in example 1 was freeze dried as active enzyme and incorporated in a standard corn soy diet at a dosage depending on the activity of the samples of 354 mg/kg for K1. The dosing being the activity dose equivalent to the dose recommendation of the RPA enzyme product of 200 mg/kg. The protease "Ronozyme ProAct" was dosed according to the dose recommendation with 200 mg/kg and Phytase was dosed with 500 FTU/kg, also according to the standard dosing recommendation.

The experiment was performed with one-day-old male broiler chickens (Cobb 500) that were allocated to four experimental groups with eight repetitions of 3 birds each. The chickens were distributed as homogenous as possible to identical stainless steel cages with three birds per cage (8 repetitions per any treatment).

The 35 d experimental period was divided into two feeding phases; a starter period from first day to day fourteen of age and a subsequent grower period from day fifteen to day thirty-five of age, respectively. The basal starter and grower diets were calculated to meet the nutrient requirements for broiler chickens recommended by the GfE with exception of slightly reduced protein, amino acids, and phosphorus contents. One group received the basal diets without test products (control). Further groups were offered diets containing the enzyme prototype "K1" the commercial enzyme product "Ronozyme ProAct" (RPA) throughout the 35 d feeding period. A further group received the phytase enzyme product "Quantum Blue 5G".

Broiler chickens had *ad libitum* access to feed (mash form) throughout the 35 d feeding period; water supplied by drinking bells was also available *ad libitum*. The trial was run without any adverse technical events (e.g. power failure, feed/water failures). The overall mortality rate amounted to 2.5 %.

The efficacy was demonstrated by productive performance (body weight, body weight gain, feed intake, feed conversion ratio) over the full feeding period, and apparent ileal digestibility measurements (ash, crude protein, crude fat, calcium, phosphorus) at the end (35 days of age).

For calculation of the individual body weight gain the following formula was used:

Average weight gain per bird for each period F - S (corrected by weight gain of died or culled chickens)

  F - Average weight of the live birds in the cage at the weighing day
  S - Average weight of the live birds in the cage at the previous weighing

The feed intake (corrected for dispersed feed) was calculated by the following formula

$$Feed\ intake\ per\ period = \frac{total\ feed\ consumed\ per\ cage}{(number\ of\ surviving\ birds\ \times\ days\ of\ the\ period) + days\ of\ died\ birds\ alive}$$

The feed conversion ratio was estimated by using the following formula:

$$Feed\ conversion\ per\ period = \frac{total\ feed\ consumed\ per\ cage}{total\ weight\ gain\ for\ the\ period\ (with\ died\ or\ culled\ chicken}$$

Apparent ileal digestibility of crude protein, crude fat, crude ash, calcium and phosphorus was determined in all birds of each treatment group at day 35 on trial (5 days of age). The ileal contents of 3 birds of one cage were pooled. Before analysing the pooled samples were kept at -20°C before being freeze-dried for chemical analyses.

Titanium(IV) oxide (TiO2) was supplemented as an inert marker at the dose level of 3 g/kg diet. For calculation of the apparent ileal digestibility the following formula was used:

$$Ileal\ digeatability\ (\%) = 100 - \left[ \frac{\%\ marker\ in\ feed}{\%\ marker\ in\ ileum} \times \frac{\%\ nutrient\ in\ ileum}{\%\ nutrient\ in\ feed} \times 100 \right]$$

**Table 3: Performance from day 1 to day 35**

| | | control (T1) | RPA (T2) | K1 (T3) | Quantum Blue (T5) |
|---|---|---|---|---|---|
| Broiler chickens | no | 23 | 24 | 24 | 23 |
| Body weight start | g | 41.6 ± 0.9 | 41.6 ± 1.1 | 41.6 ± 1.4 | 41.5 ± 1.2 |
| Body weight end | g | 2067.3 ± 25.9[a] | 2119.8 ± 34.5[b] | 2177.8 ± 34.9[c] | 2080.2 ± 26.0[ab] |
| Body weight gain | g | 2025.6 ± 26.0[a] | 2078.1 ± 34.9[b] | 2136.1 ± 35.0[c] | 2038.7 ± 25.7[ab] |
| Feed intake | g | 2951.2 ± 68.1 | 2955.8 ± 46.2 | 2971.2 ± 85.3 | 2938.7 ± 62.2 |
| Feed conversion [1)] | | 1.457 ± 0.036[b] | 1.422 ± 0.013[ab] | 1.391 ± 0.047[a] | 1.442 ± 0.033[b] |
| Points FCR increase | | | 3.5 | 6.6 | 1.5 |
| Different superscripts within lines indicate levels of significance at P < 0.05 | | | | | |

When feeding broiler chickens with inclusion of the acid protease "K1" benefits on overall body weight gain were significantly higher than those reported for "Ronozyme ProAct", or the phytase enzyme product "Quantum Blue 5G" (table 3). The corresponding overall feed conversion ratio was significantly reduced by 6.6 points compared to the control and 5.1 points to broiler chickens fed "Quantum Blue 5G". Reduction of the food conversion ratio was also 3.1 points higher than reported for "Ronozyme ProAct".

The positive response on performance of the enzymes was based on improvements in the apparent ileal digestibility measured at day 35 of age (measurements performed as described in example 10). As already reported in the feeding trial shown in example 10, the apparent ileal digestibility of crude protein was increased but also significant effects on the digestibility of calcium, phosphate and fat were observed (table 4). The averaged apparent ileal digestibility increase of the acid proteases K1 was 7,9 %., being significant higher than the observed increase for the neutral active protease Ronozyme ProAct with 4,3 %.

The significant and unexpected high effects on the apparent digestibility of phosphate (K1, 8,8 % increase to control) and calcium (K1, 12,2 % and increase to control) can be attributed to the known interaction between phytate and protein. The publication of Selle et al. (2000) consolidates the information about phytate-protein complexes and discusses the effect of phytase and the phytase-associated positive effects on protein digestibility. Under acid conditions of the stomach, below the isoelectric point of proteins, binary protein-phytate complexes are formed, whereas ternary complexes of phytate, metal ions and proteins are formed at neutral pH (Cosgrove 1966, Anderson 1985). Binary protein-phytate complexes have been demonstrated *in vitro* at acid pH for several proteins e.g. glycinin (Okubo et al. 1976) the major protein in soybeans also being a protein source in the feeding trials described above and in example 10 and 12. The maximum of protein complexation to binary complexes have been described at pH 2 - 3 for globulin, with a dependence on the phytate to protein ratio. Rajendran & Prakash (1993) described progressive protein-protein aggregation after an initial binding of protein to phytate associated with conformational changes of the protein. Such aggregates might be recalcitrant to protein hydrolysis as several *in vitro* studies describe a reduction of peptic hydrolysis in the presence of phytate in acid conditions (Camus & Laporte, 1976). This initial description was extended by several studies consistently showing a reduction of peptic activity for plant storage and animal proteins (Kanaya et al. 1976, Inagawa et al. 1987, Knuckles et al. 1989, Vaintraub & Bulmaga 1991).

The working hypothesis was derived from these observations that the formation of binary protein-phytate complexes are a major aspect of the wellknown anti-nutritive effect of phytate. The anti-nutritive effect of phytate today is only addressed by means of supplemented microbial phytase activity beside the effect of the liberation of dietary phosphate from phytate. The protein effect of phytase is most probably associated with the protein-phytate complexes, by hydrolysis of phytate to lower inositol phosphates with a rendered ability to form such complexes. It might also be that a new propagated reading of dose recommendation, the superdosing of phytase exerts its effect by fast hydrolyzing phytate and interfering with the built up of such complexes. No examples of using a protease from peptidase family S53 to address these protein-phytate complexes are described so far though it is obvious that also phytase will hydrolyse soluble phytate better (Lonnerdal et al. 1989) than phytate in protein decorated complexes (Konishi et al. 1999, Bohn et al. 2007).

Releasing protein and phytate from such binary complexes, in which phytate and protein are both more recalcitrant to hydrolysis, by the action of an acid protease, does have the potential to have synergistic effects with phytase. This effect is a result of the simultaneous activity of both enzymes and haven't been tested for neutral active proteases before or been observed in the *in vivo* trials shown in examples 10, 11 and 12. Testing the hydrolysis of binary complexes *in vitro* is hard to test as complex preparation render the complex nature and predictivity is concomitantly limited (Selle et al. 2000). *In vivo* feeding trials might be the best way to test such effects. It can be expected that an acid protease able to hydrolyze protein in binary complexes or before such complexes have been formed will have beneficial effects on the digestibility of crude protein and at least additional effects with phytase on the digestibility of phosphorous, with more than additive, i.e. synergistic effects being a good prove for the discussed mode of action and the inventive step in selecting an acid protease which is active on binary protein-phytate complexes. Otherwise picking an acid enzyme is not obvious, as acid proteases do have less time to hydrolyze protein due to a 4 time longer retention time in the neutral parts of the gastrointestinal tract, the mostly lower thermal stability, fewer examples for the economical producibility and examples for genetic engineering of such enzymes for better performance.

**Table 4: Apparent ileal digestibility at day 35**

|  |  | control (T1) | RPA (T2) | K1 (T3) | Quantum Blue (T5) |
|---|---|---|---|---|---|
| Crude Protein | digestibility % | 80.93 ± 1.36[a] | 81.95 ± 0.99[ab] | 82.53 ± 1.01[abc] | 82.67 ± 0.86[bcd] |
| Crude Fat | digestibility % | 88.52 ± 1.22[a] | 90.06 ± 0.75[abc] | 91.47 ± 0.39[bcd] | 89.74 ± 0.42[ab] |
| Ash | digestibility % | 38.87 ± 3.20[a] | 42.67 ± 1.53[ab] | 43.91 ± 2.26[bc] | 43.80 ± 1.14[bc] |
| Calcium | digestibility % | 48.93 ± 2.88[a] | 50.94 ± 2.86[ab] | 54.92 ± 2.32[bc] | 55.11 ± 3.80[bc] |

(continued)

| | | control (T1) | RPA (T2) | K1 (T3) | Quantum Blue (T5) |
|---|---|---|---|---|---|
| Phosphorous | digestibility % | 45.34 ± 2.79[a] | 47.34 ± 2.10[a] | 49.32 ± 1.78[b] | 53.54 ± 2.04[c] |
| Averaged apparent ileal digestibility increase % over control | | | 4,3 | 7,9 | 9,4 |

For better evaluation of the results exploratory graphics as box-and-whisker plots were shown in Fig 12 A- F, to show distribution of the presented datasets, for body weight gain, feed conversion and apparent ileal digestibility of crude protein, phosphorous, calcium and crude fat. Treatment groups were 1 control; 2 Ronozyme ProAct, 3 Kumamolisin AS (K1), 5 Quantum Blue 5G - each dosed as outlined above.

### Example 12: *In vivo* digestibility and performance test

To test for any synergistic effects with phytases, Kumamolisin AS (K1), as an example for an acid protease of the group of sedolisins (S53) or G1, was tested in combination with the phytase Quantum Blue (AB Vista) in an *in vivo* trial against the combination of "Ronozyme ProAct" (RPA) with the same phytase. As discussed, the latter enzyme is a protease with a neutral pH activity profile, and was included to demonstrate the performance advantage of said acid proteases of the group of sedolisins (S53) or G1, over neutral active proteolytic enzymes, when combined with a phytase.

The trial was run in parallel to and under the same conditions as outlined for the trial in example 11, with the exception that proteases were only fed from day twenty-nine to day thirty-five. Control, basal diet without enzyme (treatment 1) and the phytase reference, basal diet with phytase "Quantum blue 5G" (treatment 5) were the same as in example 11. Further groups offered diets with "Quantum blue 5G" for the full feeding period of 35 days with additional protease for the last seven days (day 29 to day 35), "Ronozyme ProAct" (treatment 6) and Kumamolisin AS (treatment 7). Further groups were protease control groups to test for the effect of protease "Ronozyme ProAct" (treatment 8) or and Kumamolisin AS (treatment 9) when only feed for the last seven days of the 35 day feeding period and in the absence of phytase.

The dosing was equal to and described in example 11. In brief, enzyme produced as described in example 1 was freeze dried as active enzyme and incorporated in the starter and grower diets outlined in example 11 at a dosage depending on the activity of the samples of 354 mg/kg for K1, being the activity dose equivalent to the dose recommendation of the RPA enzyme product. The protease "Ronozyme ProAct" was dosed according to the dose recommendation with 200 mg/kg and phytase was dosed with 500 FTU/kg, also according to the standard dosing recommendation.

The efficacy was demonstrated by productive performance (body weight, body weight gain, feed intake, feed conversion ratio) over the full feeding period, and apparent ileal digestibility measurements (ash, crude protein, crude fat, calcium, phosphorus) at the end (35 days of age). All calculations also for apparent ileal digestibility were as in example 10 and 11.

Before comparative analytics of the feeding trial results it was evaluated whether treatments feeding enzymes only for the last 7 days are relevant with regard to the overall feeding period of 35 days. By the fact that corresponding benefits (weight gain; feed conversion ratio) were found for "Ronozyme ProAct", and K1 fed throughout the 35 day feeding period or from day 29 to day 35 of age without enzyme addition before (compare treatment groups 2, 8 and 3, 9 in table 5) results of the enzyme combinations measured from day 29 to day 35 are sufficiently relevant. The fact that results on apparent ileal digestibility of "Ronozyme ProAct" and K1 were nearly independent from feeding duration (compare lines line for treatment groups T2 and T3 from table 3 in example 11, with lines from treatment group T8 and T7 in table 6 of example 12 respectively) benefits of enzyme combinations measured from day 29 to day 35 of age seemed to be relevant with regard to the overall feeding period.

### Table 5: Performance from day 29 to day 35

| | | Control (T1) | RPA (T2) | K1 (T3) | Quantum Blue (T5) | Quantum Blue + RPA (T6) | Quantum Blue + K1 (T7) | RPA (T8) | K1 (T9) |
|---|---|---|---|---|---|---|---|---|---|
| Body weight start | g | 1424,6 ± 15,0 | 1454,9 ± 21,3 | 1490,4 ± 20,8 | 1434,1 ± 16,4 | 1435,7 ± 13,8 | 1428,2 ± 19,3 | 1422,4 ± 18,4 | 1429,4 ± 18,6 |
| Body weight end | g | 2067,3 ± 25,9 | 2119,8 ± 34,5 | 2177,8 ± 34,9 | 2080,2 ± 26,0 | 2150,8 ± 19,8 | 2173,0 ± 20,5 | 2096,1 ± 28,0 | 2112,9 ± 22,3 |

(continued)

|  | | Control (T1) | RPA (T2) | K1 (T3) | Quantum Blue (T5) | Quantum Blue + RPA (T6) | Quantum Blue + K1 (T7) | RPA (T8) | K1 (T9) |
|---|---|---|---|---|---|---|---|---|---|
| Body weight gain | g | 642,6 ± 35,6 | 664,9 ± 51,3 | 687,3 ± 42,9 | 646,1 ± 25,5 | 715,1 ± 26,0 | 744,8 ± 26,7 | 676,7 ± 25,6 | 683.5 ± 20.4 |
| Feed intake | g | 1002,9 ± 41,2 | 1008,1 ± 47,2 | 1008,9 ± 37,0 | 992,8 ± 45,2 | 1017,5 ± 37,7 | 1025,3 ± 39,3 | 1003,3 ± 55,6 | 1014,6 ± 42,1 |
| Feed conversion 1) | | 1,563 ± 0,073 | 1,521 ± 0,089 | 1,472 ± 0,102 | 1,537 ± 0,062 | 1,424 ± 0,044 | 1,377 ± 0,03 | 1,490 ± 0,091 | 1,485 ± 0,073 |
| Points FCR increase | | | 4,2 | 9,1 | 2,6 | 13,9 | 18,6 | 7,3 | 7,8 |

**Table 6: Apparent ileal digestibility at day 35**

|  | | Control (T1) | RPA (T2) | K1 (T3) | Quantum Blue (T5) | Quantum Blue + RPA (T6) | Quantum Blue + K1 (T7) | RPA (T8) | K1 (T9) |
|---|---|---|---|---|---|---|---|---|---|
| Crude Protein | % | 80.93 ± 1.36 | 81.95 ± 0.99 | 82.53 ± 1.01 | 82.67 ± 0.86 | 83.83 ± 1.39 | 84.29 ± 0.81 | 82.52 ± 1.05 | 83.19 ± 0.79 |
| Crude Fat | % | 88.52 ± 1.22 | 90.06 ± 0.75 | 91.47 ± 0.39 | 89.74 ± 0.42 | 92.19 ± 1.05 | 92.03 ± 0.89 | 90.78 ± 0.598 | 91.65 ± 0.60 |
| Ash | % | 38.87 ± 3.20 | 42.67 ± 1.53 | 43.91 ± 2.26 | 43.80 ± 1.14 | 44.02 ± 2.85 | 47.09 ± 4.44 | 42.12 ± 2.86 | 43.00 ± 1.90 |
| Calcium | % | 48.93 ± 2.88 | 50.94 ± 2.86 | 54.92 ± 2.32 | 55.11 ± 3.80 | 55.37 ± 1.52 | 57.68 ± 2.75 | 54.53 ± 2.02 | 55.33 ± 2.05 |
| Phosphorous | % | 45.34 ± 2.79 | 47.34 ± 2.10 | 49.32 ± 1.78 | 53.54 ± 2.04 | 55.85 ± 2.42 | 58.51 ± 2.12 | 48.48 ± 2.89 | 49.23 ± 3.10 |
| Averaged apparent ileal digestibility increase % over control | | | 4,3 | 7,9 | 9,4 | 11,3 | 15,3 | 6,2 | 7,7 |
| Increase of phosphorus digestibility % over control | | | 2,0 | 4,4 | 8,2 | 10,5 | 13,2 | 3,1 | 3,9 |
| Increase of phosphorus digestibility % over Quantum blue | | | | | | 2,3 | 5,0 | | |
| Treatment T1 - T5 groups are reproductions from example 11 | | | | | | | | | |

The overall body weight gain for K1 was significantly higher than those recorded for chickens fed any other enzyme over the full feeding period. The corresponding overall feed conversion ratio was significantly reduced compared to the other enzymes. The highest benefits on body weight gain and feed conversion ratio were found for combinations with the phytase "Quantum Blue 5G" with effects of K1, the acid protease in combination with phytase being superior to the neutral protease "Ronozyme ProAct".

The positive response on performance of these enzymes and these enzyme combinations was based on improvements in the apparent ileal digestibility. K1 and "Ronozyme ProAct" feed for the last 7 days increased the averaged apparent digestibility by 7,7 % and 6,2 % respectively.

The averaged apparent ileal digestibility benefits were highest for combinations of the phytase "Quantum Blue 5G" and "Ronozyme ProAct" or K1 of 11,3 % or 15,3 % respectively, with K1 the acid protease combination being superior to the neutral protease "Ronozyme ProAct" combination with phytase.

The effect of combining the acid protease K1 with a phytase was better than observed for the combination of phytase with the neutral active enzyme product "Ronozyme ProAct", especially with respect to calcium and phosphorus. Also referencing the discussed potential benefits of an acid protease over a neutral protease in combination with phytase, the increase of phosphate digestibility in combination with phytase is more than additive for the acid active protease K1, pointing to synergistic effects with phytases, whereas the neutral active protease shows less than additive effects (compare table 6, line "Increase of phosphorus digestibility % over Quantum blue" for T6 and T7 to line "Increase of phosphorus digestibility % over control" for T8 and T9 respectively) .

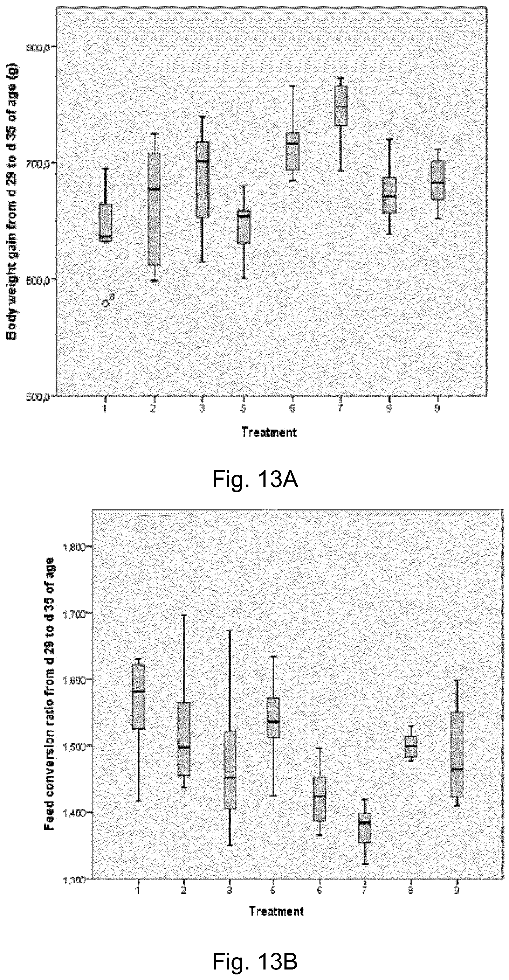

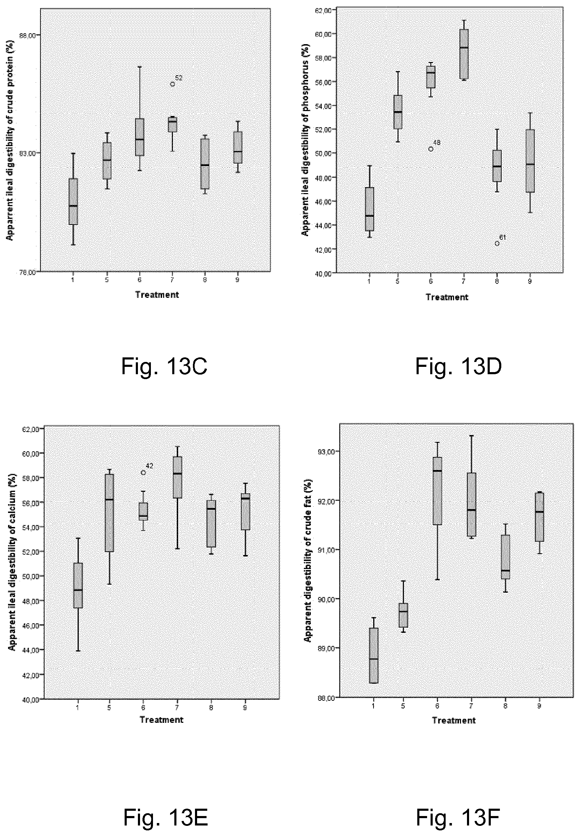

For better evaluation of the results exploratory graphics as box-and-whisker plots were shown in Fig. 13 A - F, to show distribution of the presented datasets, for body weight gain, feed conversion and apparent ileal digestibility of crude protein, phosphorous, calcium and crude fat. Treatment groups were 1 control; 2 Ronozyme ProAct, 3 Kumamolisin AS (K1), 5 Quantum Blue 5G, 6 Quantum Blue 5 G + Ronozyme ProAct (day 29 - 35), 7 Quantum Blue 5 G + K1 (day 29 - 35), 8 Ronozyme ProAct (day 29 - 35), 9 K1 (day 29 - 35) - each dosed as outlined above.

These experiments show impressively that there is a true synergism between an acid protease from the S53 family and a phytase. As discussed, acid proteases from the S53 family and the G1 family form a group of what were formerly termed "pepstatin-insensitive carboxyl proteinases". Hence, both families share a particular structural and/or functional relationship. For this reason, said synergism also applies to a combination of an acid protease from the G1 family and a phytase, when compared to a combination of a neutral/alkali protease with a phytase.

**Table 7: Performance for treatment period from day 22 to day 28**

| | | control | RPA | K1 |
|---|---|---|---|---|
| Broiler chickens | no | 24 | 24 | 24 |
| Body weight start | g | $824.3 \pm 24.7$ | $824.0 \pm 20.9$ | $824.3 \pm 23.7$ |
| Body weight end | g | $1330.0 \pm 25.2$ | $1341.4 \pm 22.7$ | $1352.1 \pm 22.9$ |
| Body weight gain | g | $505.8 \pm 19.2$ | $517.4 \pm 11.3$ | $527.9 \pm 21.5$ |
| Feed intake | g | $726.9 \pm 30.3$ | $727.8 \pm 17.3$ | $730.5 \pm 24.2$ |
| Feed conversion | (kg feed per kg body weight gain) | $1.438 \pm 0.044$ | $1.407 \pm 0.032$ | $1.385 \pm 0.032$ |
| Points FCR increase | | | 3,1 | 5,3 |
| Different superscripts within lines indicate levels of significance at P < 0.05 | | | | |

**Table 8: Apparent ileal digestibility at end of treatment period day 28**

| | | control | RPA | K1 |
|---|---|---|---|---|
| Crude Protein | digestibility % | $76.00 \pm 1.56^a$ | $78.60 \pm 0.92^b$ | $80.18 \pm 1.03^c$ |
| Crude Fat | digestibility % | $91.43 \pm 2.21^a$ | $93.99 \pm 1.17^b$ | $94.67 \pm 0.79^b$ |
| Ash | digestibility % | $36.09 \pm 4.89^a$ | $38.50 \pm 1.85^a$ | $45.11 \pm 3.76^b$ |
| Calcium | digestibility % | $35.30 \pm 4.46$ | $38.55 \pm 4.94$ | $38.31 \pm 63.68$ |
| Phosphorous | digestibility % | $45.14 \pm 2.91^a$ | $48.39 \pm 2.31^{ab}$ | $51.70 \pm 3.31^b$ |
| Averaged apparent ileal digestibility increase % over control | | | 5,86% | 11,42% |

**Example 13: Expression of grifolisin**

Grifosilin is another peptidase from the S53 family originating from the fungus *Grifola frondosa*. Grifolisin was expressed in *Saccharomyces cerevisiae* strain BY4741, transformed with a plasmid harboring the codon optimized gene (SEQ ID NO 3) under the control of a constitutive promotor as a zymogen. The enzyme was secreted via a *S. cerevisiae* specific leader into the culture medium and further processed to retain active enzyme.

The expression took place in SC-Ura media supplemented with 2 % glucose adjusted to pH 5.6. A pre-culture of 10 mL SC-Ura 2 % glucose media was inoculated with 50 $\mu$l glycerol stock containing the transformed cells and grown at 30°C 150 rpm for 24 h. For the main culture 1 L SC-Ura 2v% glucose medium were supplemented with the pre-culture adjusting to an OD600 of 0.05, and the cells were then further grown at 30°C 150 rpm for 72 h.

**Example 14: Hydrolysis of soybean derived Kunitz-type and Bowman-Birk Trypsin Inhibitors (BBI/KTI)**

In soy beans and other legume seeds, two types of trypsin inhibitors are found in soy beans: the Kunitz trypsin inhibitor (KTI) and the Bowman-Birk inhibitor (BBI). KTI is a large (20,100 daltons), strong inhibitor of trypsin, while BBI is much smaller (8,000 daltons) and inhibits both trypsin and chymotrypsin, which occur naturally in the gut of humans and livestock.

Both inhibitors have significant anti-nutritive effects in the body, affecting digestion by hindering protein hydrolysis and activation of other enzymes in the gut. Whole soybeans contain 15-40 mg of trypsin inhibitor per gram, hence, between 1,5 % w/w and 4,0 % w/w, and do hence a form a significant fraction of the bean's protein content.

The presence of these inhibitors is thought to protect soy seeds against consumption by animal predators.

Interstingly, the two inhibitors are remarkably stable, and furthermore largely resistant against digestion by proteases. In feed applications, an enzymatic degradation of these two inhibitors has a twofold effect, namely (i) makes said protein fraction accessible for uptake by the animal, and (ii) blocks inhibition of the animals own gut proteases, trypsin and chymotrypsin.

Both effects increase the food conversion rate, and enhance the protein uotake from the feedstuff.

However, many proteases, like trypsin and chmotrypsin, do not degrade neither Kunitz trypsin inhibitor (KTI) not Bowman-Birk inhibitor (BBI). In the following experiment, it has been demonstrated that another member of the peptidase family S53, Grifolisin, can effectively degrade KTI and BBI, hence leading to a recovery of normal trypsin activity.

Experimental protocol

a) Hydrolysis of KTI and BBI with grifosilin 96-well format Incubate KTI (17.5 $\mu$g/ml) and/or BBI (4.3 $\mu$g/mL) with 14 $\mu$g/ml purified grifosilin for 60 min at 37°C
Assay buffer: 100 mM Na-Citrate, 1 mM CaCl$_2$, pH 3

b) Residual activity of trypsin after BBI and KTI hydrolysis by grifosilin using AAPF assay 96-well format

Dilute reaction mix from a) 1:5 in 24 $\mu$L 100 mM Na-Citrate, 1 mM CaCl$_2$, pH 3

Dilute 1:1 Trypsin solution in 1 M Na-phosphate pH 8 (this adjusts reaction mix pH to 7) and incubate for 10 min at 37°C.

Start reaction by adding substrate working solution for protease assay (see example 2, except that assay buffer has pH 7 for neutral protease activity)

Measure kinetics at 37°C by monitoring the increase in adsorption at 410 nm as a measure for enzymatic activity. We will compare the residual activity of trypsin in the presence and absence of BBI, KTI, and BBI/KTI.

Concentrations in the protease assay:

| KTI | 1.5 $\mu$g/mL |
|---|---|
| BBI | 0.37 $\mu$g/mL |
| BBI/KTI | 0.6 + 0.3 $\mu$g/mL |
| Trypsin | 0.1 $\mu$g/mL |
| +/- Grifosilin | 1.2 $\mu$g/mL |
| AAPF | 1 mM |

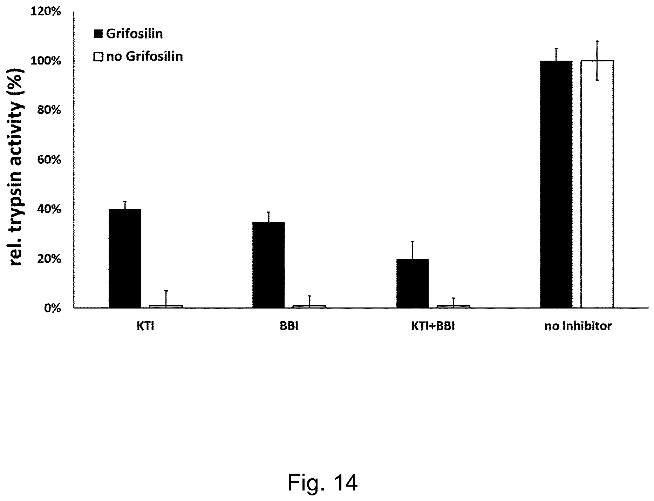

Fig. 14 clearly shows that inhibitor-mediated trypsin inhibition is strongly decreased in the presence of grifosilin, for BBI, KTI, and BBI/KTI. In numbers, 20-40% of the initial trypsin activity could be restored. Without grifosilin, no trypsin activity in presence of any type of inhibitor is visible, while in the presence of grifosilin, said activity was restored. This data

suggest that grifosilin is active on both KTI and BBI, and has hence a true effect in feed applications, where it can help to incrase the food conversion rate and the protein uptake efficiency.

Normal BBI/KTI hydrolysis assays usually show the inhibitor degradation via SDS-PAGE but never address the functional consequences of KTI/BBI hydrolysis on trypsin itself, which is the most important aspect. This functional assay here directly measures the trypsin activity after treating BBI and KTI with an acid feed protease from the S53 family, and thus, it is much closer to real-life applications.

## References

Wlodawer A1, Li M, Gustchina A, Oyama H, Dunn BM, Oda K., Acta Biochim Pol. 2003;50(1):81-102

Terashita,T., Oda,K., Kono,M. & Murao,S., Agric Biol Chem (1981) 45, 1937-1943

Oda,K., Takahashi,S., Ito,M. & Dunn,B.M., Adv Exp Med Biol (1998) 436, 349-353

Britton, H. T. K. and R. A. Robinson. J. Chem. Soc., 1931, 1456-1462.

P.H. Selle, V. Ravindran, R.A, Caldwell and W.L. Bryden. Phytate and Phytase: consequences for protein utilisation. Nutrition Research Reviews (2000), 13, p. 255-278

D.J. Cosgrove (1966). The chemistry and biochemistry of inositol polyphosphates. Reviews of pure and applied chemistry, 16, p. 209-224

P.A. Anderson (1985) Interactions between proteins and constituents that affect protein quality. In Digestibility and Amino Acid Availability in Cereals and Oilseeds, pp. 31-45 [J.W. Finley and D.T. Hopkins, editors]. St. Paul, MN: American Association of Cereal Chemistry, Inc.

K. Okubo, D.V. Meyers and G.A. Iacobucci (1976) Binding of phytic acid to glycinin. Cereal Chemistry 53, pp 513-524

Rajendran and V. Prakash (1993) Kinetics and thermodynamics of the mechanism of interaction of sodium phytate with alpha-globulin. Biochemistry 32, pp. 3474-3478

M.C. Camus and J.C. Laporte (1976) Inhibitionde la proteolyse pesique par le blé. Rôle de l'acide phytique des issues.(Inhibition of pepsin proteolysis by wheat. Role of phytic acid in the outcome). Annales de Biologie Animale Biochimie Biophysique 16, pp. 719 - 729

K. Kanaya, K. Yasumoto and H. Mitsuda (1976) Pepsin inhibition by phytate contained in rice bran. Eiyo To Shokuyo 29, pp. 341-346

J. Inagawa, I. Kiyosawa and T. Nagasawa (1987) Effects of phytic acid on the digestion of casein and soybean protein with trypsin, pancreatin and pepsin. Nippon Eiyo Shokuryo Gakkaishi 40, pp. 367-373

B.E. Knuckles, D.D. Kuzmicky, M.R. Gumbmann and A.A. Betschart (1989) Effect of myo-inositol phosphate esters on in vitro an in vivo digestion of protein. J. of Feed Science 54, pp. 1348-1350

I.A. Vaintraub and V.P. Bulmaga (1991) Effect of phytate on the in vitro activity of digestive proteinases. J. of Agricultural and Food Chemistry 39, pp. 859-861

B. Lonnerdal, A.S. Sandberg, B. Sandstrom and C. Kunz (1989) Inhibitory effects of phytic acid and other inositol phosphates on zinc and calcium absorption in suckling rats. J. of Nutrition 119, pp. 211-214

Bohn, L., Josefsen, L., Meyer, A.S., Rasmussen, S.K., 2007 Quantitative analysis of phytate globoids isolated from wheat bran and characterization of their sequential dephosphorylation by wheat phytase. J. Agri. Food Chem. 55, pp. 7547-7552

C. Konishi, T. Matsui, H. Park, H. Yano and F. Yano (1999) Heat treatment of soybean and rapeseed meals suppress rumen degradation of phytate phosphorus in sheep. Animal Feed Science and Technology 80, pp. 115-122

## Sequences

SEQ ID No 1 Kumamolisin AS codon optimized for *Bacillus subtilis*

```
tcagatatggaaaaaccgtggaaagaaggcgaagaagctagagcagttct
gcaaggccatgcaagagcacaagcaccgcaagcagttgataaaggaccgg
ttgcaggcgacgaaagaatggcagttacagttgttctgcgcagacaaaga
gcaggcgaactggcagcacatgttgaaagacaagcagcaattgcaccgca
tgcaagagaacatctgaaaagagaagcatttgcagcatcacatggcgcat
cactggatgattttgcagaactgagaagatttgcagatgcgcatggcctg
gcactggatagagcaaatgttgcagcaggcacagcagttctgtcaggacc
ggttgatgcaattaatagagcatttggcgttgaactgcgccattttgatc
atccggatggctcatatagatcatatctgggcgaagttacagttccggca
tcaattgcaccgatgattgaagcagttctgggcctggatacaagaccggt
tgcaagaccgcattttagaatgcaaagacgcgcagaaggcggatttgaag
caagatcacaagcagcagcaccgacagcatatacaccgctggatgttgca
caagcatatcaatttccggaaggcctggatggacaaggccaatgcattgc
aattattgaacttggcggaggctatgatgaagcatcactggcacaatatt
ttgcatcactgggcgttccggcaccgcaagttgtttcagtttcagttgat
ggcgcatcaaatcaaccgacaggcgatccgtcaggaccggatggcgaagt
tgaactggatattgaagttgcaggcgcactggcacctggcgcaaaatttg
cagtttattttgcaccgaatacggatgcaggctttctggatgcaattaca
acagcaattcatgatccgacactgaaaccgtcagttgtttcaattagctg
gggaggaccggaagattcatggacatcagcagcgattgcagcaatgaata
gagcgtttcttgatgcagcagcactgggcgttacagttctggcagcagca
ggcgattcaggcagcacagatggcgaacaagatggcctgtatcatgttga
ttttccggcagcatcaccgtatgttctggcatgcggaggcacaagacttg
ttgcatcaggcggaagaattgcacaagaaacagtttggaatgatggacct
gatggcggagcaacaggcggaggcgtttcaagaattttttccgcttccggc
atggcaagaacatgcaaatgttccgcccttcagcaaatcctggcgcatcat
caggcagaggcgttccggatctggcaggcaatgcagatccggcaacaggc
tatgaagttgttattgatggcgaagcgacagttattggcggaacatcagc
agttgcaccgctgtttgcagcactggttgcaagaattaatcaaaaactgg
gcaaagcagtcggctatctgaatccgacactgtatcaacttccggcagat
gtctttcatgatattacagaaggcaacaacgatattgcgaatcgcgcaca
aatttatcaagcaggaccgggatgggatccgtgcacaggcctgggctcac
cgattggcgttagactgctgcaagcactgctgccgtcagcatcacaaccg
caaccgtaa
```

**SEQ ID NO 2: Aspergillopepsin 2 codon optimized for *Saccharomyces cerevisiae***

```
gctccattgactgaaaaaagaagagctagaaaagaagctagagctgctgg
taagagacattctaatccaccatatattccaggttccgacaaagaaatct
```

tgaagttgaacggtactaccaacgaagaatactcttctaattgggctggt
gctgttttgattggtgatggttatacaaaggttaccggtgaattcactgt
tccatctgtttctgctggttcttcaggttcttctggttatggtggtggtt
acggttattggaaaaacaagagacaatccgaagaatattgtgcttctgct
tgggttggtattgatggtgatacttgtgaaactgctatcttgcaaactgg
tgttgatttctgttacgaagatggtcaaacttcttacgatgcttggtatg
aatggtatccagattacgcttacgatttctccgatattaccatctctgaa
ggtgattccatcaaggttactgttgaagctacctctaaatcatctggttc
tgccactgttgaaaacttgactactggtcaatctgttacccatactttct
ctggtaatgttgaaggtgacttgtgtgaaactaatgccgaatggatcgtt
gaagatttcgaatctggtgattctttggttgcttttgctgatttcggttc
tgttactttcactaacgctgaagctacttctggtggttctactgttggtc
catctgatgctactgttatggatattgaacaagacggttccgttttgacc
gaaacttctgtttcaggtgattctgttaccgttacttacgtttga

**SEQ ID NO 3: Grifolisin codon optimized for Saccharomyces cerevisiae**

actccaagagttccattgtccgaacaatctcatccttccaatatgatcac
ctcttctttcttggttgtctccttgtttactttggctttgtctaagccaa
tgtccagatctatgaaggttcacgaaactagagaaggtattccagatggt
tttgctttggctggttctccttcttctgatacttcttgaacttgagaat
tgccttggtccaaaatgatccagctggtttggaaactgcattatacgatg
ttaataccccatcctctgctaactacggtaaccatttgtctaaagccgaa
gttgaaaagttcgttgctccagaaccagaatctgttgatgctgttaatgc
ttggttggaagaaatggtttgactgctactactatttctcctgctggtg
attggttggcttttgaagttccagtttctaaggccaacgaattattcgat
gctgatttctctgtttacacccatactgatactggtttagaagccattag
aaccttgtcctattctattccagctgaattgcaaggtcacttggatttgg
ttcatccaactattactttcccaaacccatactcaagattgccagttgtt
gcttcttctattaagactgctgctccaacttctgataacttgacttcttt
ggctgttccatcttcttgtgcttctacaattactccagcttgtttacaag
ccttgtacggtattccaactacaccagctactcaatcttctaacaaattg
gctgtttccggttacattgaacaattcgctaatcaagccgacttgaaaac
tttcttgactaagttcagaaccgacatctcttcttctactactttcacta
ctcaaaccttggatggtggtgaaaatccacaaaatggtaatgaagctggt
gttgaagctgatttggatgttcaatatactgttggtttggctactgatgt
tccaaccgttttcatttctgttggtgataactttcaagacggtgctttgg
aaggtttcttggacattatcaatttcttgttggacgaatctaccccacct
caagttttgactacttcttatggtcaaaacgaaaacaccatctccagaaa
cttggctaacaatttgtgtaacgcttacgctcaattgggtgctagaggta
cttctattttgtttgcttcaggtgacggtggtgtttctggttcacaatct
gattcttgttctaagttcgttccaactttcccatctggttgtcctttttat
gacttcagttggtgctactacaggtattaacccagaaactgctgctgatt
tttcttctggtggtttctctaattacttcggtactccatcttatcaagcc
tctgctcattctgcttacttgcaagccttgggttctactaatgctggtaa
gtttaatacctctggtagaggttttccagacgtttctactcaaggtgaaa

```
acttccaaatcgttgttgatggtcaaaccggtacagttgatggtacatca
tgtgcttctccaacctttgcttctgttgtttctttgttgaacgatagatt
gattgctgccggtaaatctccattgggttttttgaatccattcttgtact
ctactggtgcttctgcctttaactctattacatctggttctaacccaggt
tgtaacactaatggtttcccagctaaaactggttggtcaccagttactgg
tttgggtactccaaattttgctaagttgttaaccgccgttggttta
```

**SEQ ID NO 4: Aspergillopepsin 2 codon optimized for *Hansenula polymorpha***

```
gctccattgactgaaaaaagaagagctagaaaagaagctagagctgctgg
taagagacattctaatccaccatatattccaggttccgacaaagaaatct
tgaagttgaacggtactaccaacgaagaatactcttctaattgggctggt
gctgttttgattggtgatggttatacaaaggttaccggtgaattcactgt
tccatctgtttctgctggttcttcaggttcttctggttatggtggtggtt
acggttattggaaaaacaagagacaatccgaagaatattgtgcttctgct
tgggttggtattgatggtgatacttgtgaaactgctatcttgcaaactgg
tgttgatttctgttacgaagatggtcaaacttcttacgatgcttggtatg
aatggtatccagattacgcttacgatttctccgatattaccatctctgaa
ggtgattccatcaaggttactgttgaagctacctctaaatcatctggttc
tgccactgttgaaaacttgactactggtcaatctgttacccatactttct
ctggtaatgttgaaggtgacttgtgtgaaactaatgccgaatggatcgtt
gaagatttcgaatctggtgattctttggttgcttttgctgatttcggttc
tgttactttcactaacgctgaagctacttctggtggttctactgttggtc
catctgatgctactgttatggatattgaacaagacggttccgttttgacc
gaaacttctgtttcaggtgattctgttaccgttacttacgtttga
```

## Claims

1. A feed composition comprising a protease from Peptidase family S53, which has a pH of $\geq 6$,

   wherein the composition or one or more enzymes therein has increased stability and/or storage life, compared to a composition comprising a protease from Peptidase family S53 which has a pH of $\leq 5,5$.

2. The composition according to claim 1, wherein said protease is at least one selected from the group consisting of Kumamolisin-AS (also called Kumamolisin 1), Kumamolisin-AC and/or Grifolisin.

3. The composition according to any one of the aforementioned claims, wherein said composition further comprises a phytase.

4. The composition according to any one of the aforementioned claims,

   a) which composition further comprises at least one agent or buffer that is present in a concentration suitable to maintain the pH of said composition at a value of $\geq 6$, or
   b) wherein the pH of $\geq 6$ is caused by the protein expression as such, or by the cultivation conditions or fermentation conditions.

5. The composition according to any one of the aforementioned claims, which composition comprises at least one further enzyme.

6. A method of activating a composition according to any one of the aforementioned claims, which method comprises decreasing the pH of said composition to a value of $\leq 5$ or smaller.

7. The method according to claim 6, wherein the decrease of the pH is at least partly accomplished by

• adding a suitable agent or buffer to the composition,
• adding the composition to another composition that has a more acidic pH, and/or
• allowing the composition to decrease its pH by means of natural processes.

8. A feed additive, a feed ingredient, a feed supplement, or a feedstuff which comprises a composition according to any of to any one of claims 1 - 5.

9. A feedstuff comprising the feed additive, ingredient, or supplement according to claim 8, or the composition according to any one of claims 1 - 5, which has a crude protein content of between $\geq 10$ and $\leq 500$ g/kg (1 - 50 % w/w), preferably comprising the protease in an amount from $\geq 0.0005$ % to $\leq 0.5$ % w/w..

10. A method of decreasing a population of bacteria in the upper gastrointestinal tract of a subject, the method comprising administering to the subject a composition according to any one of claims 1 - 5, wherein the population of bacteria is reduced in the upper gastrointestinal tract of the subject.

11. A method for improving feed efficiency, the method comprising modifying a standard diet to contain less protein, and supplementing the modified diet with the feed additive, ingredient, supplement or feedstuff according to any one of claims 8 - 9, or the composition according to any one of claims 1 - 5.


**Patentansprüche**

1. Futtermittelzusammensetzung umfassend eine Protease aus der Peptidase-Familie S53, die einen pH-Wert von $\geq 6$ hat,

wobei die Zusammensetzung oder ein oder mehrere darin enthaltene Enzyme eine erhöhte Stabilität und/oder Lagerfähigkeit aufweisen,
im Vergleich zu einer Zusammensetzung, die eine Protease aus der Peptidase-Familie S53 enthält, die einen pH-Wert von $\leq 5,5$ hat.

2. Zusammensetzung nach Anspruch 1, wobei die Protease mindestens eine ausgewählt aus der Gruppe bestehend aus Kumamolisin-AS (auch Kumamolisin 1 genannt), Kumamolisin-AC und/oder Grifolisin ist.

3. Zusammensetzung nach einem der vorgenannten Ansprüche, wobei die Zusammensetzung ferner eine Phytase umfasst.

4. Zusammensetzung nach einem der vorgenannten Ansprüche,

a) wobei die Zusammensetzung ferner mindestens ein Mittel oder einen Puffer umfasst, das in einer Konzentration vorhanden ist, die geeignet ist, den pH-Wert der Zusammensetzung auf einem Wert von $\geq 6$ zu halten, oder
b) wobei der pH-Wert von $\geq 6$ durch die Proteinexpression als solche oder durch die Kultivierungs- oder Fermentationsbedingungen verursacht wird.

5. Zusammensetzung nach einem der vorgenannten Ansprüche, welche Zusammensetzung mindestens ein weiteres Enzym umfasst.

6. Verfahren zum Aktivieren einer Zusammensetzung nach einem der vorgenannten Ansprüche, wobei das Verfahren das Absenken des pH-Werts der Zusammensetzung auf einen Wert von $\leq 5$ oder weniger umfasst.

7. Verfahren nach Anspruch 6, wobei das Absenken des pH-Wertes zumindest teilweise erreicht wird durch

• Hinzufügen eines geeigneten Mittels oder Puffers zu der Zusammensetzung,
• Hinzufügen der Zusammensetzung zu einer anderen Zusammensetzung, die einen saureren pH-Wert hat, und/oder
• Zulassen, dass die Zusammensetzung ihren pH-Wert durch natürliche Prozesse absenkt.

8.  Futtermittelzusatz, Futtermittelbestandteil, Futtermittelergänzung oder Futtermittel, das eine Zusammensetzung nach einem der Ansprüche 1 bis 5 enthält.

9.  Futtermittel, das den Futtermittelzusatz, -bestandteil oder -ergänzung nach Anspruch 8 oder die Zusammensetzung nach einem der Ansprüche 1 bis 5 enthält, das einen Rohproteingehalt zwischen $\geq 10$ und $\leq 500$ g/kg (1 bis 50 Gew.-%) hat, vorzugsweise die Protease in einer Menge von $\geq 0,0005$ % bis $\leq 0,5$ Gew.-% enthält.

10. Verfahren zur Verringerung einer Bakterienpopulation im oberen Gastrointestinaltrakt eines Subjekts, wobei das Verfahren die Verabreichung einer Zusammensetzung nach einem der Ansprüche 1 bis 5 an das Subjekt umfasst, wobei die Bakterienpopulation im oberen Gastrointestinaltrakt des Subjekts verringert wird.

11. Verfahren zur Verbesserung der Futtermitteleffizienz, wobei das Verfahren die Modifizierung eines Standardfutters, so dass es weniger Protein enthält, und die Ergänzung des modifizierten Futters mit dem Futtermittelzusatz, -bestandteil oder -ergänzung oder dem Futtermittel nach einem der Ansprüche 8 bis 9 oder der Zusammensetzung nach einem der Ansprüche 1 bis 5 umfasst.

**Revendications**

1.  Composition alimentaire comprenant une protéase de la famille des Peptidases S53, qui a un pH $\geq 6$,

    dans laquelle la composition ou une ou plusieurs enzymes en son sein présente(nt) une stabilité et/ou une durée de conservation accrues,
    en comparaison à une composition comprenant une protéase de la famille des Peptidases S53 qui a un pH $\leq 5,5$.

2.  Composition selon la revendication 1, dans laquelle ladite protéase est au moins une protéase sélectionnée dans le groupe constitué de Kumamolisin-AS (également appelée Kumamolisin 1), Kumamolisin-AC et/ou Grifolisin.

3.  Composition selon l'une quelconque des revendications précédentes, dans laquelle ladite composition comprend en outre une phytase.

4.  Composition selon l'une quelconque des revendications précédentes,

    a) laquelle composition comprend en outre au moins un agent ou tampon qui est présent à une concentration appropriée pour maintenir le pH de ladite composition à une valeur $\geq 6$, ou
    b) dans laquelle le pH $\geq 6$ est causé par l'expression protéique en tant que telle, ou par les conditions de culture ou les conditions de fermentation.

5.  Composition selon l'une quelconque des revendications précédentes, laquelle composition comprend au moins une enzyme supplémentaire.

6.  Procédé d'activation d'une composition selon l'une quelconque des revendications précédentes, lequel procédé comprend la diminution du pH de ladite composition à une valeur $\leq 5$ ou plus faible.

7.  Procédé selon la revendication 6, dans lequel la diminution du pH est au moins partiellement réalisée par

    • l'ajout d'un agent ou tampon approprié à la composition,
    • l'ajout de la composition à une autre composition ayant un pH plus acide, et/ou
    • le fait de permettre à la composition de diminuer son pH au moyen de processus naturels.

8.  Additif alimentaire, ingrédient alimentaire, complément alimentaire ou aliment qui comprend une composition selon l'une quelconque des revendications 1 à 5.

9.  Aliment comprenant l'additif alimentaire, l'ingrédient ou le complément selon la revendication 8, ou la composition selon l'une quelconque des revendications 1 à 5, qui a une teneur en protéines brutes comprise entre $\geq 10$ et $\leq 500$ g/kg (1 à 50 % p/p), comprenant de préférence la protéase en une quantité allant de $\geq 0,0005$ % à $\leq 0,5$ % p/p.

10. Procédé de réduction d'une population de bactéries dans la partie supérieure du tractus gastro-intestinal d'un sujet, le procédé comprenant l'administration au sujet d'une composition selon l'une quelconque des revendications 1 à 5, dans lequel la population de bactéries est réduite dans la partie supérieure du tractus gastro-intestinal du sujet.

11. Procédé pour améliorer l'efficacité alimentaire, le procédé comprenant la modification d'un régime alimentaire standard pour contenir moins de protéines, et la complémentation du régime modifié avec l'additif alimentaire, l'ingrédient, le complément ou l'aliment selon l'une quelconque des revendications 8 à 9, ou la composition selon l'une quelconque des revendications 1 à 5.

A

B

C

Fig. 1

Fig. 2

Fig. 3

Fig. 4

Fig. 5

Fig. 6

Fig. 7

Fig. 8

Fig. 9

Fig. 10

Fig. 11

Fig. 12A

Fig. 12B

Fig. 12C

Fig. 12D

Fig. 12E

Fig. 12F

Fig. 13A

Fig. 13B

Fig. 13C

Fig. 13D

Fig. 13E

Fig. 13F

Fig. 14

EP 3 475 420 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9017667 B
- WO 2016062855 A
- WO 2016062857 A

### Non-patent literature cited in the description

- **A.O.A.C**. Official Methods of Analysis. Association of Official Analytical Chemists, 1984
- **WLODAWER A1** ; **LI M** ; **GUSTCHINA A** ; **OYAMA H** ; **DUNN BM** ; **ODA K.** *Acta Biochim Pol.*, 2003, vol. 50 (1), 81-102
- **TERASHITA,T.** ; **ODA,K** ; **KONO,M.** ; **MURAO,S.** *Agric Biol Chem*, 1981, vol. 45, 1937-1943
- **ODA,K** ; **TAKAHASHI,S.** ; **ITO,M.** ; **DUNN,B.M.** *Adv Exp Med Biol*, 1998, vol. 436, 349-353
- **BRITTON, H. T. K.** ; **R. A. ROBINSON.** *J. Chem. Soc.*, 1931, 1456-1462
- **P.H. SELLE** ; **V. RAVINDRAN** ; **R.A, CALDWELL** ; **W.L. BRYDEN.** Phytate and Phytase: consequences for protein utilisation. *Nutrition Research Reviews*, 2000, vol. 13, 255-278
- **D.J. COSGROVE**. The chemistry and biochemistry of inositol polyphosphates. *Reviews of pure and applied chemistry*, 1966, vol. 16, 209-224
- Interactions between proteins and constituents that affect protein quality.. **P.A. ANDERSON**. In Digestibility and Amino Acid Availability in Cereals and Oilseeds. American Association of Cereal Chemistry, Inc, 1985, 31-45
- **K. OKUBO** ; **D.V. MEYERS** ; **G.A. IACOBUCCI**. Binding of phytic acid to glycinin. *Cereal Chemistry*, 1976, vol. 53, 513-524
- **RAJENDRAN** ; **V. PRAKASH**. Kinetics and thermodynamics of the mechanism of interaction of sodium phytate with alpha-globulin. *Biochemistry*, 1993, vol. 32, 3474-3478
- **M.C. CAMUS** ; **J.C. LAPORTE**. Inhibitionde la proteolyse pesique par le blé. Rôle de l'acide phytique des issues.(Inhibition of pepsin proteolysis by wheat. Role of phytic acid in the outcome).. *Annales de Biologie Animale Biochimie Biophysique*, 1976, vol. 16, 719-729
- **K. KANAYA** ; **K. YASUMOTO** ; **H. MITSUDA**. Pepsin inhibition by phytate contained in rice bran.. *Eiyo To Shokuyo*, 1976, vol. 29, 341-346
- **J. INAGAWA** ; **I. KIYOSAWA** ; **T. NAGASAWA**. Effects of phytic acid on the digestion of casein and soybean protein with trypsin, pancreatin and pepsin. *Nippon Eiyo Shokuryo Gakkaishi*, 1987, vol. 40, 367-373
- **B.E. KNUCKLES** ; **D.D. KUZMICKY** ; **M.R. GUMBMANN** ; **A.A. BETSCHART**. Effect of myo-inositol phosphate esters on in vitro an in vivo digestion of protein. *J. of Feed Science*, 1989, vol. 54, 1348-1350
- **I.A. VAINTRAUB** ; **V.P. BULMAGA**. Effect of phytate on the in vitro activity of digestive proteinases. *J. of Agricultural and Food Chemistry*, 1991, vol. 39, 859-861
- **B. LONNERDAL** ; **A.S. SANDBERG** ; **B. SANDSTROM** ; **C. KUNZ**. Inhibitory effects of phytic acid and other inositol phosphates on zinc and calcium absorption in suckling rats. *J. of Nutrition*, 1989, vol. 119, 211-214
- **BOHN, L** ; **JOSEFSEN, L.** ; **MEYER, A.S.** ; **RASMUSSEN, S.K**. Quantitative analysis of phytate globoids isolated from wheat bran and characterization of their sequential dephosphorylation by wheat phytase. *J. Agri. Food Chem.*, 2007, vol. 55, 7547-7552
- **C. KONISHI** ; **T. MATSUI** ; **H. PARK** ; **H. YANO** ; **F. YANO**. Heat treatment of soybean and rapeseed meals suppress rumen degradation of phytate phosphorus in sheep.. *Animal Feed Science and Technology*, 1999, vol. 80, 115-122